# EUROPEAN PATENT APPLICATION

(11) **EP 4 296 302 A1**
(43) Date of publication of application: **27.12.2023**
(21) Application number: 22305924.7
(22) Date of filing: 24.06.2022
(51) Int. Cl.: C08J 11/12, C07C 67/333, C07C 69/54, C10B 53/07, C10B 57/06, C10G 1/10, B01D 5/00, C10B 47/00, B01D 1/14, B01D 1/16, C10K 1/02

(54) **DEPOLYMERIZATION METHOD AND SYSTEM**

(71) Applicant: ARKEMA FRANCE, 92700 Colombes (FR); Heathland B.V., 3565 AR Utrecht (NL); Japan Steel Works Europe GmbH, 40217 Düsseldorf (DE)
(72) Inventor: DUBOIS, Jean-Luc, 92700 Colombes (FR); HEESINK, Bert, 7512EE Enschede (NL); VERBEEK, Erik, 7512EE Enschede (NL); KONING, Bert, 7512EE Enschede (NL); VAN DER HEIJDEN, Simon, 3565AR Utrecht (NL); TOJO, Makoto, 40217 Düsseldorf (DE)
(74) Representative: Lavoix

(57) **Abstract**

The depolymerization method comprises the successive steps of pyrolizing a feed material containing polymer in a pyrolysis reactor so as to generate a hot gas stream, removing solid and liquid impurities from the gas stream in a separator and condensing the monomer contained in the gas stream in a condenser.

The gas stream is transferred from the separator to the condenser via a transfer column which is provided with internals. Cold monomer in liquid state is injected into the transfer column, the internals being configured such that the cold monomer flows downwardly inside the transfer column by gravity, heavy contaminants contained in the gas stream condense in liquid state on the internals and flow back down by gravity towards the bottom of the transfer column and into the separator.

## Description

The project leading to this application has received funding from the European Union's Horizon 2020 research and innovation program under grant agreement No 820687.

The present invention relates to the field of depolymerization for recovering a monomer from a feed material containing a polymer from which the monomer is a constituent.

It relates more particularly to a thermal depolymerization method comprising the pyrolysis of the feed material, i.e. the heating of the feed material such as to vaporize the feed material at least in part, in a substantial absence of oxygen. The pyrolysis of the feed material generates a gas stream including vapor of the monomer.

A problem arising in such a depolymerization method is that the gas stream generated by the pyrolysis may contain impurities such as dust, mist droplets, remaining polymer chains, oligomers and/or contaminants, in addition to the vaporized monomer.

Such impurities may alter components of the depolymerization system located downstream the pyrolysis reactor, thus negatively impacting the productivity and the cycle time between cleaning, or contaminate the monomer, retrieved e.g. as crude monomer or oil monomer, obtained from the vaporized monomer.

JP3210323 and DE3146194 each disclose a depolymerization method for recovering a monomer from a polymer comprising the steps of pyrolizing a feed material containing the polymer into a pyrolysis reactor such as to generate a gas stream containing monomer vapor and passing the gas stream through a separator such as to remove impurities from the gas stream.

WO2016/030460 discloses a depolymerization method for recovering a monomer from a polymer comprising the steps of pyrolizing a feed material containing the polymer into a pyrolysis reactor such as to generate a gas stream containing monomer vapor and passing the gas stream through a contactor containing perforated plates arranged for flow of long chain components back to the pyrolysis reactor.

US10731080 discloses a depolymerization method for recovering a monomer from a polymer comprising the steps of pyrolizing a waste material containing the polymer to generate a gas stream, quenching the gas stream output from the pyrolysis reactor in a quenching apparatus for removing oligomers from the gas stream and routing the oligomers back to the pyrolysis reactor.

One of the aims of the invention is to propose a depolymerization method that allows producing monomer, in particular crude monomer or pyrolysis oil, with a satisfying quality and with a satisfying productivity.

To the end, the invention proposes a depolymerization method for recovering a monomer from a polymer, the depolymerization method comprising the successive steps of pyrolizing a feed material containing the polymer in a pyrolysis reactor so as to generate a hot gas stream, removing solid and liquid impurities from the gas stream in a separator and condensing the monomer contained in the gas stream in a condenser,
wherein the gas stream is transferred from the separator to the condenser via a transfer column extending upwardly from a lower end fluidly connected to an outlet of the separator to an upper end fluidly connected to an inlet of the condenser,
wherein cold monomer in liquid state is injected into the transfer column, the transfer column being provided with internals configured such that the cold monomer flows downwardly inside the transfer column by gravity, heavy contaminants contained in the gas stream condense in liquid state on the internals and flow back down by gravity towards the bottom of the transfer column with being then transferred to the separator and the temperature of the gas stream decreases from the lower end to the upper end of the transfer column, and
wherein the temperature of the gas stream at the outlet of the separator and the inlet of the transfer column is maintained above the boiling point of the monomer and the temperature of the gas stream at the outlet of the transfer column is maintained between the boiling point of the monomer and the boiling point of the monomer + 50°C.

In specific embodiments, the depolymerization method comprises one or several of the following optional features, taken individually or in any technically feasible combination:
- the temperature of the gas stream at the outlet of the separator and entrance of the transfer column is maintained at least at the boiling point of the monomer + 20°C, in particular at least at the boiling point of the monomer + 30°C, more in particular at least at the boiling point of the monomer + 40°C, even more in particular at least at the boiling point of the monomer + 50°C;
- the temperature of the gas stream at the outlet of the transfer column is maintained between the boiling point of the monomer and the boiling point of the monomer + 50°C, in particular between the boiling point of the monomer and the boiling point of the monomer + 40°C, in particular between the boiling point of the monomer and the boiling point of the monomer + 30°C, in particular between the boiling point of the monomer and the boiling point of the monomer + 20°C, in particular between the boiling point of the monomer and the boiling point of the monomer + 10°C;
- the internals comprise one mesh filter or a plurality of mesh filters arranged in cascade inside the transfer column;
- the internals comprise a structured packing and/or a random packing, for example a random packing including saddles and/or rings, in particular Pall rings;
- the internals comprise a succession of baffles;
- the internals comprise a succession of baffles comprising internally heated baffles, preferably alternating with non-heated baffles;
- cold monomer in liquid state is injected at the upper end of the transfer column and/or at one or several intermediate locations between the lower end and the upper end of the transfer column;
- it comprises the step of filtering the cold monomer before injection of the cold monomer into the transfer column;
- it comprises a step of inputting thermal energy inside the transfer column and/or in the separator by heating the gas and/or the liquid present inside the transfer column and/or inside the separator;
- it comprises heating liquid collected in the separator using a heater located inside the separator and/or heater located outside the separator for evaporating monomer contained in the liquid and returning the monomer to the separator;
- heating of the liquid is performed:
   - with a heater provided as an electrical heater arranged outside the separator for heating liquid collected inside the separator;
   - with a heater provided as an evaporator, in particular a wiped film evaporator, preferably an evaporator arranged inside the separator, the evaporator being fed with liquid collected from the separator; and/or
   - with a heater provided as a heated screw extruder fed with liquid collected from the separator, a gas produced by the heater provided as a heated screw extruder being fed to the separator.
- the rate of cold monomer injected in the transfer column is at least 50%, preferably at least 60%, preferably at least 70% preferably at least 80%, preferably at least 90% of the equivalent feed rate of the monomer;
- the fraction of cold monomer injected in the transfer column is less than 153%, preferably less than 103%, preferably less than 93% preferably less than 83%, preferably less than 73%, preferably less than 63%, preferably less than 53% of the equivalent feed rate of the monomer;
- the temperature of the gas stream at the inlet of the separator is equal or higher than 250°C;
- the temperature of the gas stream at the outlet of the separator is comprised between 100 °C and 280 °C, preferably between 110°C and 180°C;
- the pressure inside at least one or each of the pyrolysis reactor, the separator and the condenser is comprised between 0.3 to 2.0 bars, preferably between 0.4 and 1.5 bars, more preferably between 0.5 and 1.2 bars.

The invention also relates to a depolymerization system configured for recovering a monomer from a polymer, the depolymerization system comprising a pyrolysis reactor configured for pyrolizing a feed material containing the polymer so as to generate a gas stream, a separator fluidly connected to the pyrolysis reactor for receiving the gas stream from the pyrolysis reactor and configured for removing impurities from the gas stream, and a condenser fluidly connected to the separator for receiving the gas stream from the separator and configured for condensing the monomer contained in the gas stream received from the separator, wherein the depolymerization system comprises a transfer column for transferring the gas stream from the separator to the condenser, the transfer column extending upwardly from a lower end fluidly connected to an outlet of the separator to an upper end fluidly connected to an inlet of the condenser, the transfer column being configured for injection of cold monomer in liquid state inside the transfer tube and provided with internals configured such that in operation the cold monomer flows downwardly inside the transfer tube by gravity, heavy contaminants contained in the gas stream deposit in liquid state on the internals and flow back by gravity towards the separator and the temperature of the gas stream increases from the lower end to the upper end of the transfer column.

The invention and its advantages will be better understood upon reading the following description given solely by way of non-limiting example and with reference to the appended drawings in which:
- Figure 1 is a schematic diagram illustrating a depolymerization system for implementing a depolymerization method for recovering a monomer from a feed material containing a polymer from which the monomer is a constituent;
- Figure 2 is a diagrammatic side view of a separator that may be used in the depolymerization system of Figure 1;
- Figure 3 is a diagrammatic top view of an alternative separator of Figure 2;
- Figure 4 is a diagrammatic exploded side view of the separator of Figure 3;
- Figure 5 is a diagrammatic side view of a separator;
- Figure 6 is a diagrammatic side view of a separator;
- Figure 7 is a diagrammatic top view of a separator and a primary separator;
- Figure 8 is a diagrammatic side view of the primary separator;
- Figure 9 is a diagrammatic exploded view of a separator;
- Figure 10 is a diagrammatic side view of a the depolymerization system illustrating more particularly a transfer column having a lower end fluidly connected to the outlet of the separator and an upper end fluidly connected to an inlet of the condenser, the transfer column having internals comprising packings;
- Figure 11 is a diagrammatic side view of a transfer column having internals comprising grids, filters or perforated plates;
- Figure 12 is a diagrammatic side view of a transfer column having internals comprising baffles;
- Figure 13 is a diagrammatic side view of a transfer column having internals comprising baffles including jacketed baffles;
- Figure 14 is a diagrammatic side view of a separator including a thin film evaporator for heating liquid collected from the separator and returned to the separator.
- Figure 15 is a schematic diagram illustrating the use of a reheating extruder used for reheating liquid collected from the separator and to be returned to the separator.

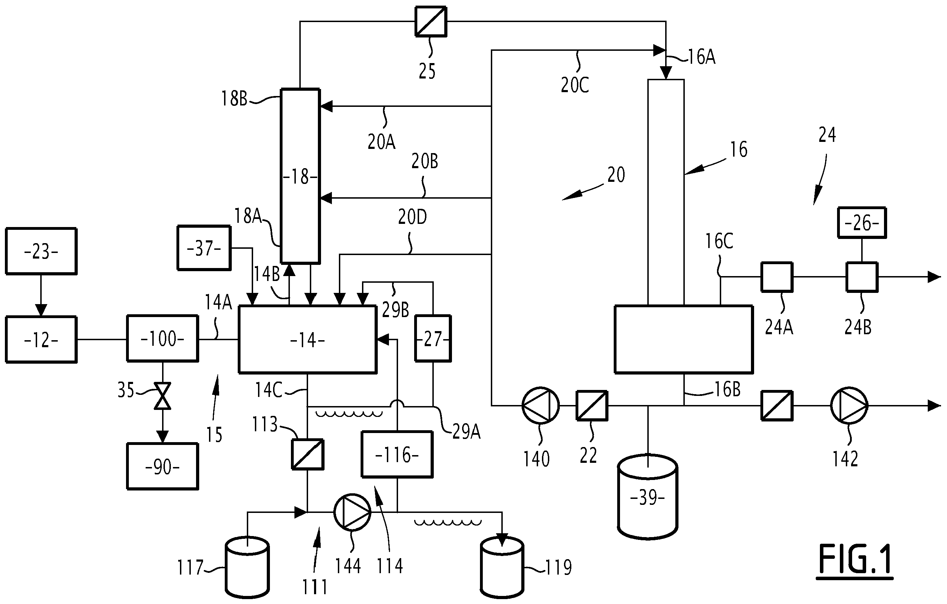

The depolymerization system 10, illustrated on Figure 1, is configured for implementing a depolymerization method for recovering a monomer from a feed material containing a polymer from which the monomer is a constituent.

The depolymerization system 10 comprises a pyrolysis reactor 12 configured for generating a gas stream from a feed material containing the polymer, a separator unit 15 comprising a separator 14 configured for removing liquid and solid impurities from the gas stream, a condenser 16 configured for condensing the monomer in gaseous state contained in the gas stream into monomer in liquid state and a transfer column 18 configured for transferring the gas stream from the separator 14 to the condenser 16.

The pyrolysis reactor 12, the separator 14, the transfer column 18 and the condenser 16 are fluidly connected in series in this order.

The depolymerization method comprises the successive steps of pyrolizing the feed material in the pyrolysis reactor 12 for generating a gas stream from a feed material containing the polymer, the gas stream containing the monomer (which is a constituent of the polymer) in gaseous state, directing the gas stream from the pyrolysis reactor 12 to the separator 14 for removing liquid and solid impurities from the gas stream, and transferring the gas stream from the separator 14 to the condenser 16 for condensing the monomer in gaseous state contained in the gas stream into monomer in liquid state.

During implementation of the depolymerization method, the pyrolysis is operated such that the gas stream provided to the separator 14 contains the monomer in gaseous state, which is also referred to as "gaseous monomer" in the following.

During implementation of the depolymerization method, the condensate provided by the condenser 16 contains the monomer, or pyrolysis oil, in liquid state also referred to as "liquid monomer" or "cold monomer" in the following.

According to the depolymerization method, the transfer of the gas stream from the separator 14 to the condenser 16 is performed via the transfer column 18 which extends upwardly from a lower end 18A fluidly connected to a gas outlet 14B of the separator 14 to an upper end 18B fluidly connected to a gas inlet 16A of the condenser 16

According to the depolymerization method, cold monomer in liquid state is injected into the transfer column 18, the transfer column 18 being provided with internals configured such that the cold monomer flows downwardly inside the transfer column 18 by gravity, heavy contaminants contained in the gas stream flowing upwardly through the transfer column 18 condense in liquid state on the internals and flow back down by gravity towards the lower end 18A of the transfer column 18, with being then transferred to the separator 14, the temperature of the gas stream decreasing from the lower end 18A to the upper end 18B of the transfer column 18.

The depolymerization system 10 comprises for example a reinjection loop 20 configured for collecting liquid monomer from the condenser 16, more particularly from a condensate outlet 16B of the condenser 16, and injecting this liquid monomer in the transfer column 18, in particular at the upper end 18B of the transfer column 18.

As illustrated on Figure 1, the reinjection loop 20 comprises a reinjection line 20A for reinjecting the liquid monomer at the upper end 18B of the transfer column 18.

Optionally, the reinjection loop 20 is configured for reinjecting the liquid monomer collected from the condenser 16 at one or several intermediate injection points located between the lower end 18A and the upper end 18B of the transfer column 18, at a gas inlet 16A of the condenser 16 and/or in the separator 14.

As illustrated on Figure 1, the reinjection loop 20 comprises a reinjection line 20B for reinjecting the liquid monomer at an intermediate point along the transfer column 18, a reinjection line 20C for reinjecting the liquid monomer at the gas inlet 16A of the condenser 16 and/or a reinjection line 20D for reinjecting the liquid monomer in the separator 14.

Optionally, the depolymerization method comprises filtering the cold monomer retrieved from the condenser 16 before reinjection of the cold monomer into the transfer column 18 and optionally the condenser 16 and/or the separator 14.

As illustrated on Figure 1, the reinjection loop 20 comprises a filtering device 22 for filtering the liquid monomer retrieved from the condenser 16 before reinjection in the transfer column 18 and optionally the condenser 16 and/or the separator 14. The filtering device 22 comprising for example on filter or two filters fluidly connected in parallel.

According to the depolymerization method, the temperature of the gas stream at the separator gas outlet 14B of the separator 14 and the lower end 18A of the transfer column 18 is maintained above the boiling point of the monomer and the temperature of the gas stream at the upper end 18B of the transfer column 18 is maintained between the boiling point of the monomer and the boiling point of the monomer + 50°C.

The depolymerization method is for example operated such that the temperature of the gas stream at the gas outlet 14B of the separator 14 and the lower end 18A of the transfer column 18 is for example maintained at least at the boiling point of the monomer + 20°C, in particular at least at the boiling point of the monomer + 30°C, more in particular at least at the boiling point of the monomer + 40°C, even more in particular at least at the boiling point of the monomer +50°C.

Preferably, the depolymerization method is for example operated such that the temperature of the gas stream at the upper end 18B of the transfer column 18 is maintained between the boiling point of the monomer and the boiling point of the monomer + 50°C, in particular between the boiling point of the monomer and the boiling point of the monomer + 40°C, in particular between the boiling point of the monomer and the boiling point of the monomer + 30°C, in particular between the boiling point of the monomer and the boiling point of the monomer + 20°C, in particular between the boiling point of the monomer and the boiling point of the monomer + 10°C.

Preferably, according to the depolymerization method, the rate of cold monomer injected in the transfer column 18 is at least 50%, preferably at least 60%, preferably at least 70% preferably at least 80%, preferably at least 90% of the equivalent feed rate of the monomer generated by the depolymerization.

The equivalent feed rate of the monomer is defined as the flow rate of pyrolized monomer generated by depolymerization and entering the inlet 14A of the separator 14.

Preferably, the fraction of cold monomer retrieved from the condenser 16 and reinjected in the transfer column 18 is less than 153%, preferably less than 103%, preferably less than 93% preferably less than 83%, preferably less than 73%, preferably less than 63%, preferably less than 53% of the equivalent feed rate of the monomer.

Preferably, the depolymerization method is carried out such that the temperature of the gas stream at the inlet 14A of the separator 14 is equal or higher than 250°C, preferably equal or higher than 300 °C, still preferably equal or higher than 350°C, more preferably equal or higher than 400 °C, and even more preferably equal or higher than 420 °C.

The temperature of the gas stream at the inlet 14A of the separator 14 is in particular a function of the operation of the pyrolysis reactor 12 that is configured for heating the feed material as this will be explained after.

Preferably, the depolymerization method is operated such that the temperature of the gas stream at the outlet 14B of the separator 14 is comprised between 100 and 280°C, preferably between 110 and 180 °C.

Preferably, the depolymerization method is operated such that the pressure inside the pyrolysis reactor 12, the separator 14, the transfer column 18 and the condenser 16 is comprised between 0.3 to 2.0 bars, preferably between 0.4 and 1.5 bars, more preferably between 0.5 and 1.2 bars.

The pyrolysis reactor 12 comprises a reactor inlet 12A for receiving the feed material and a reactor outlet 12B for providing the gas stream resulting from the pyrolysis of the feed material.

The depolymerization system 10 comprises for example a feeding device 23 for feeding material containing the polymer to the pyrolysis reactor 12, in particular to the reactor inlet 12A. The feeding device 23 comprises at least one feeder. The feeding device 23 comprises for example one feeder or preferably at least two feeders feeding the pyrolysis reactor 12 in parallel.

The pyrolysis reactor 12 is configured for heating the feed material received at the reactor inlet 12A at high temperature such as to vaporize the feed material to generate the gas stream provided at the reactor outlet 12B.

Optionally, the pyrolysis reactor 12 is configured for mechanically processing the feed material. Mechanically processing of the feed material in addition to heating the feed material promotes generating the gas stream.

Mechanically processing the feed material in the pyrolysis reactor 12 may for example include stirring and/or extruding the feed material. Hence, the pyrolysis reactor 12 is for example configured for stirring the feed material and/or extruding the feed material.

The pyrolysis reactor 12 is for example configured as a heating screw extruder, in particular a heated twin-screw extruder.

A heating screw extruder comprises a tubular barrel, at least one screw extending inside the barrel with being driven in rotation for extruding the feed material along the tubular chamber, and a heating system for heating the feed material contained in the barrel.

A heating twin-screw extruder is a heating screw extruder comprising two screws extending side-by-side in the barrel, the threads of the screw being preferably intermeshed, the screws being either co-rotating (rotating in the same direction about their respective axes) or counter-rotating (rotating in opposed directions about their respective axes).

The separator unit 15 has an internal flow passage for the gas stream. The gas stream flows through the separator unit 15 via the internal flow passage thereof.

The separator 14 comprises a separator gas inlet 14A for receiving the gas stream generated by the pyrolysis reactor 12 and a separator gas outlet 14B for providing the gas stream to be transferred to the condenser 16 via the transfer column 18.

Optionally, the separator 14 comprises at least one impurities outlet 14C for collecting impurities separated from the gas stream in the separator 14.

The separator 14 has an internal flow passage for the gas stream from the separator gas inlet 14A to the separator gas outlet 14B. The internal flow passage of the separator 14 has an upstream section adjacent the separator gas inlet 14A and a downstream section adjacent the separator gas outlet 14B.

The internal flow passage of the separator 14 defines at least a section of the internal flow passage of the separator unit 15.

The internal flow passage of the separator unit 15 (including that of the separator 14 or consisting of that of the separator 14) has a cross-section taken substantially perpendicularly to the flow of gas inside the internal flow passage. The cross-section has an area (i.e. the measure of the extent of the cross-section).

The area of the cross-section of the internal flow passage of the separator unit 15 or the separator 14 may be constant along the internal flow passage or vary along the internal flow passage.

In the following, the "largest cross-section" of the internal flow passage of the separator unit 15 or the separator 14 refers to the section of the internal flow passage of the separator unit 15 or the separator 14 having the cross-section that as the largest area.

The condenser 16 comprises the gas inlet 16A for receiving the gas stream provided by the separator 14 and the condensate outlet 16B for providing the condensate, i.e. the products that have condensed in the condenser 16.

The condenser 16 is configured for condensing the gas stream received at the gas inlet 16A and provide a condensate at the condensate outlet 16B.

The condenser 16 optionally comprises a gas outlet 16C for providing non-condensate products that pass through the condenser 16 without condensing.

The depolymerization method is preferably implemented such that the gas stream in the separator 14 is at a temperature that is equal to or higher than the boiling temperature of the monomer.

When the inlet flow passage is provided, the gas stream produced by the pyrolysis travels along the inlet flow passage of the separator 14 with cooling progressively. The temperature of the gas stream in the separator 14 is the lowest in the downstream section of the separator 14.

In such case, the depolymerization method is preferably implemented such that the temperature of the gas stream in the downstream section of the separator 14 is equal to or higher than the boiling temperature of the monomer.

This will promote evaporation of any liquid monomer present in the gas stream into gaseous monomer and this will prevent polymerization of the monomer.

The gas stream may contain dimer or trimer, i.e. chains of two monomers or three monomers. Such dimer or trimer may be due to incomplete depolymerization during pyrolysis or to oligomerization or polymerization of the monomer.

Generally, the boiling temperature of dimer and trimer of a monomer is strictly higher than the boiling temperature of the monomer.

Preferably, the depolymerization method is implemented such that the temperature of the gas stream in the downstream section of the separator 14 is at a temperature that is strictly inferior to the boiling temperature of the trimer of the monomer and/or inferior to the boiling temperature of the dimer of the monomer.

This will cause most of the dimer and/or trimer present in the gas stream to condensate in the separator 14 thus retaining dimer and trimer in the separator 14 and limiting or avoiding them to flow to the transfer column 18 and to condenser 16.

The depolymerization method is for example implemented with polymethyl methacrylate (PMMA) which refers to a homo- or copolymer of methyl methacrylate (MMA) that comprises at least 50%, preferably at least 60%, more preferably at least 70%, advantageously at least 80% and more advantageously at least 90% by weight of methyl methacrylate.

In the case of a PMMA (polymer) and MMA (monomer), the boiling temperature of the MMA is approx. 100°C and the boiling temperature of the dimers and the trimers are approx. 200°C and 300 °C respectively, at atmospheric pressure.

Hence, in such case, the depolymerization method is implemented such that the temperature of the gas stream at the separator outlet 14B and the lower end 18A of the transfer column 18 is higher than 100°C.

Besides, preferably, the depolymerization method is implemented such that the temperature of the gas stream in the downstream section of the inlet flow passage of the separator 14 is comprised between 100°C and 280°C, in particular between 110 and 180 °C.

Advantageously, the depolymerization method comprises spraying into the separator 14 liquid monomer that has been retrieved from the condenser 16, the liquid monomer being sprayed into the gas stream, and preferably facing the gas stream.

Optionally, the depolymerization method comprises spraying a fraction of the liquid monomer recovered by the condenser 16 into the separator 14.

Spraying liquid monomer recovered by the condenser 16 into the separator 14 provides a cleaning action in the separator 14, e.g. on walls of the separator 14 or on demisting devices provided inside the separator 14.

Preferably, the spraying of the liquid monomer recovered by the condenser 16 into the separator 14 is performed intermittently, e.g. during cleaning phases.

In such case, the depolymerization system 10 is configured for spraying a fraction of the liquid monomer recovered by the condenser 16 into the separator 14, e.g. via the reinjection line 20D of the reinjection loop 20.

Preferably, liquid monomer is sprayed into the separator 14 such that the ratio of the mass rate of liquid monomer sprayed into the separator 14 to the mass rate of gas stream is equal or inferior to 93%, still preferably equal or inferior to 83%, still preferably equal of inferior to 73%, still preferably equal or inferior to 63%, still preferably equal or inferior to 53%.

Preferably, the liquid monomer is sprayed into the separator 14 such that the mass rate of liquid monomer sprayed into the separator 14 to the mass rate of gas stream is comprised equal or superior to 10%, still preferably equal or superior to 20%, preferably equal or superior to 30%, still preferably equal or superior to 40%, still preferably equal or superior to 50%.

Optionally, as an alternative or a complement to the spraying of liquid monomer inside the separator 14, the depolymerization method comprises spraying liquid water inside the separator 14. The benefit is similar to that of spraying liquid monomer inside the separator 14.

The pyrolysis reactor 12 is configured for processing the feed material with a maximum mass flow rate and thus for providing the gas stream with a maximum mass flow rate.

Further, in any section of the internal flow passage of the separator unit 15, the gas stream flowing in the separator unit 15 has a linear velocity that is determined as the volume of gas produced by the pyrolysis reactor 12 by time unit divided by the area of the cross-section of said section of the internal flow passage of the separator unit 15.

Preferably, the depolymerization method is implemented such that the linear velocity of the gas stream in the separator unit 15 is lower than a maximum linear velocity at least in the section of the internal flow passage of the separator unit 15 having the largest cross-section.

In a particular embodiment, the section of the internal flow passage of the separator unit 15 having the largest cross-section is a downstream section of the internal flow passage of the separator unit 15.

In one embodiment, in particular if the polymer is PMMA, the maximum linear velocity is of 0.50 m/s, preferably 0.15 m/s, in particular of 0.10 m/s.

The linear velocity of the gas stream in any section of the internal flow passage of the separator unit 15 is a function of the mass flow rate of the gas stream or the volume flow rate of the gas stream and of the area of the cross-section of said section of the internal flow passage of the separator unit 15. The mass flow rate is related to the volume flow rate and the mass density of the gas stream at the operating pressure.

The volume flow rate of the gas stream is a function of the feed rate of the pyrolysis reactor 12, the depolymerization temperature of the polymer and the molar weight of the polymer.

Advantageously, the area of the largest cross-section of the internal flow passage of the separator unit 15 is for example chosen as a function of the maximum feed rate of the pyrolysis reactor 12, the depolymerization temperature of the polymer and the molar weight of the polymer such that the linear velocity of the gas stream in the largest cross-section of the internal flow passage of the separator unit 15 is lower than the maximum linear velocity at the maximum feed rate of the pyrolysis reactor.

Preferably, the largest cross-section of the internal flow passage of the separator unit 15 has an area that is at least 0.001 times the maximum mass flow rate of the gas stream provided by the pyrolysis reactor 12, the area being expressed in square meters (m²) and the maximum mass flow rate being expressed in kilograms per hour (kg/h).

Preferably, the area of the largest cross-section of the internal flow passage of the separator unit 15is at least ten times the area of the smallest cross-section in the separator unit 15.

These parameters allow limiting the linear velocity of the gas stream in the separator unit 15, thus favoring deposition of dust and droplets carried by the gas stream and thus separation of these droplets from the gas stream. Indeed, the lower the linear velocity of the gas stream, the less easily droplets and dusts are carried by the gas stream.

Advantageously, the depolymerization method comprises slowing down the gas stream progressively inside the separator unit 15.

This is obtained e.g. by progressively increasing a cross-section of an internal flow passage of the separator 14 from the separator gas inlet 14A to the separator gas outlet 14B.

In such case, the downstream section of the internal flow passage of the separator 14 is the section of the internal flow passage of the separator 14 having the largest cross-section.

Advantageously, the residence time of the gas stream in the separator unit 15 is comprised between 1 s and 30 s, preferably between 5 s and 25 s, still preferably between 10 s and 20 s.

The residence time is the time that is necessary for gas stream to flow through the separator unit 15.

The residence time is a function of the length of the internal flow passage of the separator unit 15, the average temperature, the mass flowrate or the volume flowrate and the molecular weight, and so of the internal volume of the separator unit 15.

The gas stream generated by the pyrolysis reactor 12 may contain a mist formed of mist droplets. Mist droplets may form around dust particles present in the gas stream. Dust particle may be due to impurities in the feed material containing the polymer that is intended to be depolymerized. Mist droplets present in the gas steam may comprise liquid monomer, liquid dimer or liquid trimer carried onto dust particles present in the gas stream.

Advantageously, the depolymerization method comprises demisting the gas stream (e.g. with passing the gas stream through a demister or demisting device) and/or desublimating the gas stream (i.e. with passing the gas stream through a desublimator or desublimation device).

Demisting is different from condensing monomer in gaseous state to monomer in liquid state. Demisting consists in collecting droplets in liquid state that are already present in the gas stream, like droplets in a fog.

Demisting is obtained for example by favoring coalescence of mist droplets present in the gas stream, e.g. by defining small passages forcing the droplets to flow closer to each other and then to coalesce.

Demisting is performed e.g. by providing at least one demisting device, each demisting device being configured for favoring coalescence of the mist droplets such that the mist droplets become too heavy for being transported by the gas stream.

Each demisting device may comprise a cyclone effect chamber, at least one grid, at least one mesh, at least one piece of foam, at least one porous monolith and/or at least one baffle for capturing mist droplets, e.g. by favoring coalescence of the mist droplets.

Optionally, the depolymerization method comprises demisting the gas stream inside the separator 14.

In such case, the separator 14 comprises an internal demisting device located inside the separator 14.

Optionally, the depolymerization method comprises demisting the gas stream downstream the condenser 16, i.e. the gas stream collected from the condenser gas outlet 16C of the condenser 16.

In such case, the depolymerization system 10 comprises a demisting unit 24 comprising at least one downstream demisting device 24A, 24B arranged downstream the condenser 16, each downstream demisting device 24A, 24B being configured for removing droplets present in the gas stream.

The demisting unit 24 comprises for example two demisting devices 24A, 24B arranged serially on a fluid line collecting gas from the condenser gas outlet 16C of the condenser 16.

The upstream demisting device 24A is for example configured for demisting the gas at the temperature of the gas, and the downstream demisting device 24B is for example configured for cooling and demisting the gas at the same time, using for example cold provided by a cold generator 26.

Desublimation differs from demisting as it provides retaining a gaseous part of a gas stream as solids.

Desublimation comprises passing the gas stream through a chamber containing lamellas or fins which are cooled such that the product to be separated from the rest of the gas stream desublimates around or below its triple point and adheres to lamellas or fins in crystalline form.

The desublimator progressively charges with the separated product in crystalline or solid form and can be drained by heating so that the crystallized or amorphous solid separated product melts. It requires that the separated product to be trapped can be heated to melt without polymerizing.

In the case of depolymerization of PMMA, providing a desublimator could be appropriate to remove Terephthalic acid of DimethylTerephthalate that could be generated by PET contamination and present in the gas stream.

Providing a desublimator is appropriate, for example in case there is a risk of contamination with polyolefin, like PE, which can generate waxes that have a high melting point and that can be trapped in the condenser 16.

A desublimation unit may comprises two desublimators fluidly connected in parallel, such that one of the two desublimators can be maintained in operation while the other is drained, thus allowing continuous operation of the depolymerization system 10.

Optionally, the depolymerization method comprises passing the gas stream through a desublimator to trap contaminants present in the gas stream.

In case a desublimation unit is provided, the latter is preferably installed serially between the separator 14 and the condenser 16.

As illustrated on Figure 1, the depolymerization system 10 optionally comprises a desublimation unit 25 provided between the separator 14 and the condenser 16 to process the gas stream flowing from the separator 14 to the condenser 16.

The desublimation unit 25 comprises for example one desublimator or two desublimators fluidly connected in parallel between the separator 14 and the condenser 16. Each desublimator can process the gas stream when the other desublimator is being discharged.

Optionally, the depolymerization system 10 comprises a side vessel 27 fluidly connected to the separator 14 such as to indicate the level of liquid in the separator 14.

The side vessel 27 is for example fluidly connected to the separator 14 via a liquid line 29A for collecting liquids from a bottom of the separator 14, the liquid connection line 29A being preferably heated, and optionally via a gas line 29B for returning gases to the separator 14.

Optionally, the depolymerization method comprises performing a primary separation between the pyrolysis reactor 12 and the separator 14, for separating dust from the gas stream before feeding the gas stream to the separator 14.

Correspondingly, the separator unit 15 optionally comprises a primary separator 100 interposed serially between the pyrolysis reactor 12 and the separator 14 and configured for separating dust from the gas stream generated by the pyrolysis reactor 12 before entry of the gas stream into the separator 14.

The depolymerization system 10 preferably comprises a solid particle collector 90 connected to the primary separator 100 for collecting the dust.

The solid particle collector 90 is for example removably connected to the primary separator unit 100, preferably via a valve 35 configured to allow swapping with another solid particle collector 90 on the go, i.e. during operation of the depolymerization system 10.

The primary separator 100 has to be taken into account for determining the ratio between the largest cross-section of the internal passage of the separator unit 15 and the maximum flow rate of the gas stream provided by the pyrolysis reactor 12 and for determining the residence time of the gas stream in the separator unit 15.

Hence, preferably, the residence time of the gas stream in the separator unit 15 (including the separator 14 and, if applicable, the primary separator 100), is comprised between 1 s and 30 s, preferably between 5 s and 25 s, still preferably between 10 s and 20 s.

Besides, preferably, the largest cross-section of the internal flow passage of the separator unit 15 (including the separator 14 and, if applicable, the primary separator 100) has an area that is at least 0.001 times the maximum mass flow rate of the gas stream provided by the pyrolysis reactor 12, the area being expressed in square meters (m²) and the maximum mass flow rate being expressed in kilograms per hour (kg/h).

Optionally, the depolymerization system 10 comprises an inert gas feeding assembly 37 configured for feeding inert gas such as nitrogen or argon into the depolymerization system 10 during a shutdown of the depolymerization system 10, and to keep a lower oxygen partial pressure in the unit.

The inert gas feeding assembly 37 is for example configured for injecting the inert gas into the separator 14.

The depolymerization system 10 preferably comprises pumps for forcing the flow of fluids in the depolymerization system 10. As illustrated on Figure 1, the depolymerization system 10 comprises for example a pump arranged at the condensate outlet 16B of the condenser 16, a pump on the reinjection loop 20 and/or a pump on the reheating loop 11.

Optionally, the depolymerization method comprises, e.g. during a cleaning phase or a starting phase, injecting liquid monomer retrieved from a liquid monomer reservoir at the upper end 18B of the transfer column 18, at one or several intermediate injection points located between the lower end 18A and the upper end 18B of the transfer column 18, at the condenser inlet 16A and/or in the separator 14.

To this end, the depolymerization system 10 comprises for example a liquid monomer reservoir 39 fluidly connected to the reinjection loop 20 for providing liquid monomer during a cleaning phase or a starting phase.

The depolymerization system 10 comprises the required pumps 140, 142, 144 that are necessary for forcing the fluids to flow through the components, lines and loops of the depolymerization system 10.

As illustrated on Figure 1, a pump 140 is provided on the reinjection loop 20, a pump 142 is provided on the condensate outlet 16B for the produced liquid monomer. A pump 144 is also provided on a reheating loop that will be details later.

A fraction of the cold monomer retrieved at the condensate outlet 16B defines a "produced" cold monomer that is collected to be used as a final product or to be further processed, in particular to be distilled. The produced cold monomer is here pumped by the pump 142.

A fraction of the cold monomer retrieved at the condensate outlet 16B is collected by the reinjection loop 20 for reinjection in the depolymerization system 10, in particular at the upper end 18B of the transfer column 18.

Preferably, the depolymerization method comprises injecting a stabilizer in the produced cold monomer.

A stabilizer is configured for preventing the cold monomer to polymerize during storage or further processing such as distillation.

Optionally, the depolymerization method comprises injecting a stabilizer in the reinjected cold monomer.

The stabilizer injected in the reinjected cold monomer is for example the same as the stabilizer injected in the produced cold monomer produced, with the same dosage or a different dosage, or is different from the stabilizer injected in the produced cold monomer.

In case the stabilizer injected in the produced cold monomer and the stabilizer injected in the reinjected cold monomer are the same, this stabilizer is for example injected in the condensate outlet 16B upstream the branching of the reinjection loop 20, whereby the dosage is the same, or the stabilizer is injected on the one hand in the condensate outlet 16B downstream the branching of the reinjection loop 20 and, on the other hand, in the reinjection loop 20, whereby the dosage is the same or different dosages are used.

In case different stabilizers are used, one stabilizer is injected in the condensate outlet 16B downstream the branching of the reinjection loop 20 and the other stabilizer is injected in the reinjection loop 20.

The stabilizer injected in the produced cold monomer is for example chosen to from one of the following families: phenothiazines, hydroquinones, topanol and butylated hydroxytoluene (or "BHT").

The stabilizer injected in the reinjected cold monomer is preferably from the family of phenothiazines or any other stabilizer that can be used in absence of oxygen.

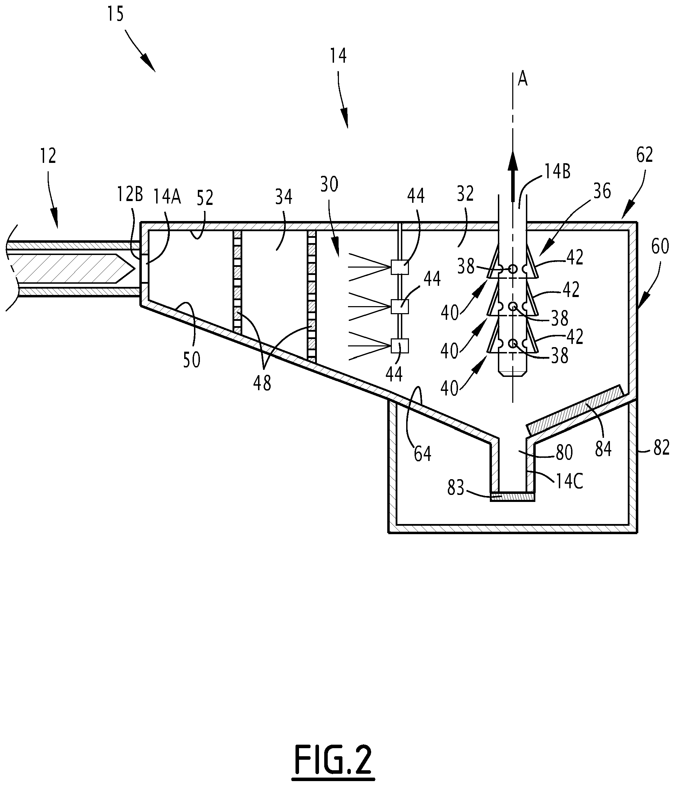

A separator 14 suitable for use in the depolymerization system 10 for implementing the depolymerization method is illustrated on Figure 2.

The separator 14 comprises the internal flow passage 30 configured for circulation of the gas stream between the separator inlet 14A and the separator outlet 14B.

In one example, as illustrated on Figure 2, the pyrolysis reactor outlet 12B opens directly into the separator inlet 14A such that the gas stream generated by the pyrolysis reactor 12 flows directly from the pyrolysis reactor outlet 12B.

Preferably, when the pyrolysis reactor 12 has at least one extruding screw, the extruding screw is inserted or protrudes into the separator inlet 14A in order to avoid blockage of the reactor outlet 12B by impurities.

In other words, the pyrolysis reactor outlet 12B is directly fluidly connected to the separator inlet 14A, such that the gas stream generated by the pyrolysis reactor 12 flows directly from the pyrolysis reactor outlet 12B connected directly to the separator inlet 14A without passing by any other fluid conducting element.

The separator 14 comprises, for example, a separation chamber 32 extending around a central axis A, the separator 14 being configured such that the gas stream flows in the separation chamber 32 circularly around the central axis A.

The separator 14 is thus configured for implementing a cyclone effect in the separation chamber 32.

With such a configuration, the gas stream flows faster at the periphery of the separation chamber 32 and slower at the center of the separation chamber 32 whereby impurities present in the gas stream tend to stay at the periphery of the separation chamber 32.

The separator 14 comprises for example an inlet duct 34, configured for receiving the gas stream generated by the pyrolysis reactor 12. The inlet duct 34 extends from the separator inlet 14A that defines the upstream end of the inlet duct 34. The inlet duct 34 forms the upstream section of the internal volume 30 of the separator 14.

In view of promoting the circular flow of the gas stream in the separation chamber 32, the inlet duct 34 emerges for example into the separation chamber 32 such as to impart to the gas stream in the separation chamber 32 a rotational movement around the central axis A.

In a particular embodiment, the inlet duct 34 opens into the separation chamber 32 such that the gas stream emerges in the separation chamber 32 along an inlet direction that defines a non-zero angle with a radial direction that is radial relative to the central axis A.

In a variant in which the separator 14 has no inlet duct 34, the separator inlet 14A opens for example into the separation chamber 32 such as to impart to the gas stream in the separation chamber 32 a rotational movement around the central axis A.

In a particular embodiment, the separator inlet 14A opens for example into the separation chamber 32 such that the gas stream emerges in the separation chamber 32 along an inlet direction that defines a non-zero angle with a radial direction that is radial relative to the central axis A.

Advantageously, the inlet duct 34 extends around the central axis A. This allows obtaining a cyclone or rotating effect in the inlet duct 34

The separator 14 is preferably configured for collecting the gas stream at the center of the separation chamber 32 and for providing the gas stream collected at the center of the separation chamber 32 to the separator outlet 14B.

The separator 14 comprises, for example, a collecting tube 36 located at the center of the separation chamber 32, the collecting tube 36 extending along the central axis A and having at least one inlet opening 38 for the gas stream present in the separation chamber 32 to enter the collecting tube 36, the collecting tube 36 being fluidly connected to the separator outlet 14B for providing the collected gas stream to the separator outlet 14B.

The collecting tube 36 is for example provided with at least one inlet section 40, each inlet section 40 comprising one inlet opening 38 or several inlet openings 38 distributed circumferentially around the inlet section 40 of the collecting tube 36.

The collecting tube 36 is for example provided with a plurality of inlet sections 40 distributed along the collecting tube 36, each inlet section 40 comprising one inlet opening 38 or several inlet openings 38 distributed circumferentially around the inlet section 40 of the collecting tube 36.

In a preferred embodiment, the collecting tube 36 is provided with at least one frustoconical baffle 42, each frustoconical baffle 42 having an upper edge of smaller diameter connected to the collecting tube 36 and a lower edge of larger diameter that is free, the baffle 42 extending around a respective inlet section 40.

Preferably, each inlet section 40 is associated with a respective frustoconical baffle 42 extending around this inlet section 40.

Preferably, the upper edge of each frustoconical baffle 42 is located above each inlet opening 38 of the corresponding inlet section 40, the lower edge of said frustoconical baffle 42 being located below each inlet opening 38 of the corresponding inlet section 40

Each frustoconical baffle 42 forces the gas stream to flow below the lower edge of the frustoconical baffle 42 before entering the inlet opening(s) 38 of the corresponding inlet section 40.

Each frustoconical baffle 42 favors impurities to fall at the bottom of the separation chamber 32 and/or to deposit onto the frustoconical baffle 42.

Preferably, each inlet opening 38 has an area comprised between 3 and 79 mm², and preferably between 12 and 51 mm²

Preferably, each inlet opening 38 has a circular contour with a diameter comprised between 2 and 10 mm, preferably between 4 and 8 mm.

Preferably, the cumulated area of the inlet openings 38 (i.e. the sum or the area of all the inlet openings 38) is comprised between 0.01 and 1.0 time the area of the cross-section of the upstream section of the internal flow passage 30 of the separator 14, in particular between 0.01 and 0.1 times the area of the cross-section of the upstream section of the internal flow passage 30 of the separator 14.

In the separator 14 of Figure 2, the upstream section of the internal flow passage 30 of the separator 14 corresponds to the upstream end of the inlet duct 34.

Advantageously, the separator 14 is configured such that largest cross-section of the separator 14 is located in the downstream section of separator 14.

In the separator 14 as illustrated on Figure 2, the downstream section of the separator 14 is defined by the separation chamber 32.

In one example in which the separator 14 comprises the inlet duct 34 and the separation chamber 32, the inlet duct 34 has a cross-section that increases progressively from the upstream end of the inlet duct 34 receiving the gas stream to the downstream end of the inlet duct 34 opening into the separation chamber 32.

The gas stream is thus slowed-down progressively in the inlet duct 34 until the gas stream reaches the separation chamber 32.

The inlet duct 34 has for example a height that increases progressively in the downstream direction and/or a width that increases progressively along the inlet duct 34 in the downstream direction such as to increase the area of the cross-section of the inlet duct 34 in the downstream direction.

Advantageously, as illustrated on Figure 2, the separator 14 is configured for spraying liquid monomer in the gas stream inside separator 14.

The separator 14 is configured for spraying one or several spray jets in the gas stream inside the separator 14.

To this end, the separator 14 comprises at least one spraying nozzle 44 for spraying liquid monomer inside the separator 14. Each spraying nozzle 44 is configured for spraying a spray jet.

The direction of each spray jet can be parallel or perpendicular or oblique to the gas flow. In one example, each spray jet is facing the gas flow, i.e. sprayed towards the upstream of the gas stream.

Each spraying nozzle 44 is fluidly connected to the condenser 16, more specifically to the return line 20D.

Each spraying nozzle 44 is arranged for example to spray the liquid monomer in the separation chamber 32.

As illustrated on Figure 2, the separator 14 comprises for example spraying nozzles 44 arranged to spray the liquid monomer at the entry of the separation chamber 32, i.e. where the inlet duct 34 connects to the separation chamber 32, here in the upstream direction of the gas stream.

Advantageously, the separator 14 integrates an internal demisting device 46 located inside the separator 14 for deposition of mist droplets transported by the gas stream.

The internal demisting device 46 comprises for example at least one grid 48 extending across a cross-section of the separator 14 such that the gas stream flows through the openings of the grid 48.

In one example in which the separator 14 comprises the inlet duct 34, each grid 48 is for example located inside the inlet duct 34.

As illustrated on Figure 2, the separator 14 comprises two grids 48 arranged in the inlet duct 34. The two grids 48 are located at a distance from each other along the inlet duct 34.

The inlet duct 34 is delimited between a lower surface 50 and an upper surface 52.

In one example, the duct lower surface 50 is inclined and/or the duct upper surface 52 is inclined.

In particular, the duct lower surface 50 is inclined and/or the duct upper surface 52 is inclined such that the cross-section of the inlet duct 34 increases from the upstream end of the inlet duct 34 towards the downstream end of the inlet duct 34.

As illustrated on Figure 2, the duct lower surface 50 is inclined such that it descends from the upstream end of the inlet duct 34 towards the downstream end of the inlet duct 34. Mist droplets depositing on the duct lower surface 50 will tend to flow towards the separation chamber by gravity.

In one example, the inlet duct 34 extends around the separation chamber 32, on at least a portion of the circumference of the separation chamber 32.

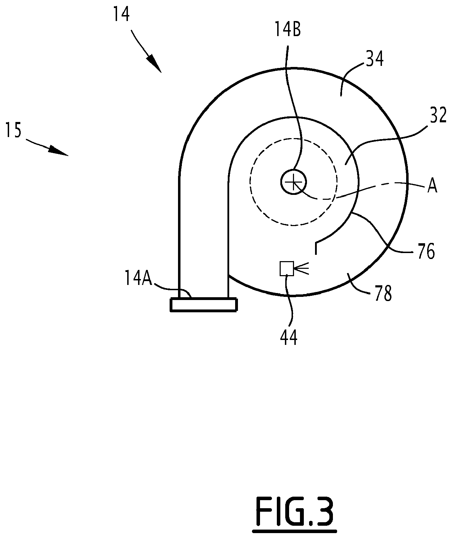

As illustrated on Figure 3, the inlet duct 34 extends around the separation chamber 32 on approximately 270° around the central axis A.

In operation, the gas stream is hot when entering the separator 14 and the temperature of the gas stream tends to lower when travelling through the separator 14 due to thermal loss, even if the separator 14 is properly thermally insulated. If spraying of liquid monomer into the separator 14 is provided, this spraying tends to further cool the gas stream in the separator 14 due to vaporization of the liquid monomer that is sprayed into the hot gas flow.

The inlet duct 34 partially surrounding the separation chamber 32 is beneficial for maintaining the gas stream sufficiently hot in the separator 14 in particular in the separation chamber 32.

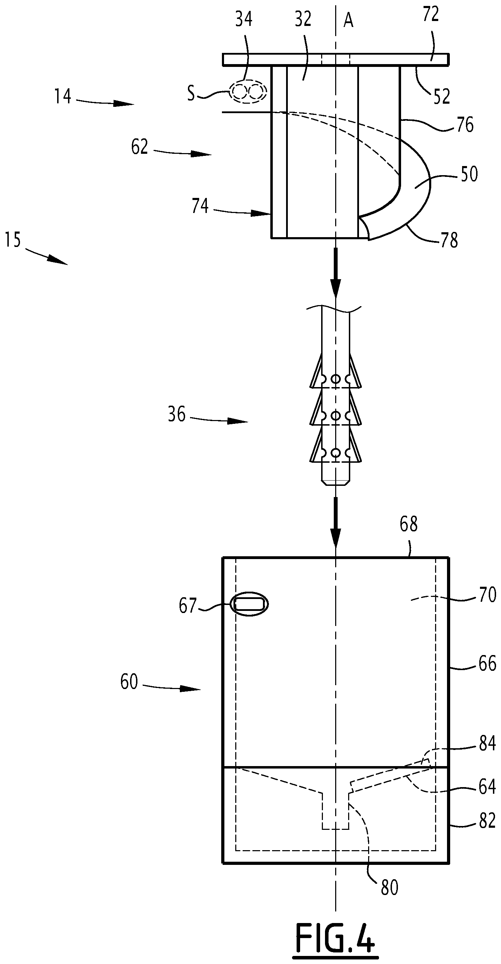

As illustrated on Figure 4, in one exemplary embodiment, the separator 14 comprises a bottom part 60 and a top part 62 to be fitted to the bottom part 60 for forming the separator 14.

The bottom part 60 comprises a bottom 64 and a peripheral wall 66 extending upwardly from the periphery of the bottom 64, the peripheral wall 66 defining an upper opening 68 and an internal volume 70. The peripheral wall 66 is provided with a side opening defining the inlet opening 14A of the separator 14. The peripheral wall 66 is for example cylindrical and of circular cross-section.

The top part 62 comprises a lid 72 for closing the upper opening 68 and an internal structure 74 extending downwardly from the lid 72 for fitting inside the bottom part 60 upon closing the upper opening 68 with the lid 72. The internal structure 74 is configured to define the separation chamber 32 and the inlet duct 34 inside the internal volume 70 of the bottom part 60.

The internal structure 74 comprises a partition wall 76 extending downwardly from the lid 72 along the central axis A for internally delimiting the separation chamber 32 and externally delimiting the inlet duct 34 separated by the peripheral wall 66, and a duct element 78 extending around the partition wall 76 for delimiting the lower surface 50 of the inlet duct 34, the upper surface 52 of the inlet duct 34 being delimited by a peripheral region of the lower face of the lid 72.

The partition wall 76 has an inner face 74A defining a lateral wall of the separation chamber 34 and an outer face 76B defining a lateral wall of the inlet duct 34.

In view along the central axis A, the partition wall 76 extends around the central axis A, approximately on 270° on Figure 3.

The inlet duct 34 is delimited between the partition wall 76, the peripheral wall 66, the duct element 78 and the lid 72.

The peripheral wall 66 is provided with a connector 67 for connection to the outlet of the pyrolysis reactor 12. The contour of the connector 67 is represented in dotted line as S to show its position relative to the upstream end of the inlet duct 34.

The lid 72 is perpendicular to the central axis A and the duct element 78 extends around the central axis A with a top surface of the duct element 78 being inclined relative to the central axis A such that the height of the inlet duct 34 increases progressively from the upstream end of the inlet duct 34 to the outlet end of the inlet duct 34, and the cross-section of the inlet duct 34 increases progressively from the upstream end of the inlet duct 34 to the outlet end of the inlet duct 34.

The duct element 78 extends for example helically around the central axis A of the separation chamber 32.

In view along the central axis A, the partition wall 76 extends for example along a portion of a circle or along a portion of a spiral with getting closer to the central axis A in the downstream direction of the inlet duct 34 delimited by the partition wall 76.

The circular extension of the partition wall 76 is used e.g. for defining an inlet duct 34 of constant width in the downstream direction of the inlet duct 34 and the spiral extension of the partition wall 76 is used e.g. for defining an inlet duct 34 of progressive increasing width in the downstream direction of the inlet duct 34.

The bottom 64 of the bottom part 60 defines the lower surface of the separation chamber 32.

The bottom 64 is provided with an evacuation opening 80 for evacuation of the impurities separated from the gas stream in the separator.

Preferably, the bottom 64 is shaped for the impurities to fall by gravity towards the evacuation opening 80. As illustrated on Figure 4, the bottom 64 is frustoconical with tapering downwardly to the evacuation opening 80.

Optionally, the separator 14 comprises a collection pot 82 that is configured for being fitted under the bottom part 60 for collecting impurities falling into the evacuation opening 80.

Preferably, the collection pot 82 is removably connected to the bottom 64. Hence, the collection pot 82 can be removed for emptying the collection pot 82.

Optionally, the evacuation opening 80 is equipped with a valve 83, for example a slide valve, arranged for selectively closing or opening the evacuation opening 80. This allows disconnecting the collection pot 82 after closing the valve 83 while the separator 14 is still in operation.

The collection pot 82 may be equipped with a gas purging line and/or the collection pot 82 may be actively cooled to allow safely removing it and replacing it.

In one example, the separator 14 is provided with a scrapping device configured for scrapping products deposited in the separator 14 to an evacuation opening. The scrapping device is configured for example for scrapping a bottom of the separator 14.

As illustrated on Figures 2 and 4, a scrapping device 84 is for example configured for scrapping products depositing on the bottom wall 64 of the bottom part 60 towards the evacuation opening 80.

The separator 14 is not limited to the example illustrated on Figures 2 - 4, other variants or options being possible.

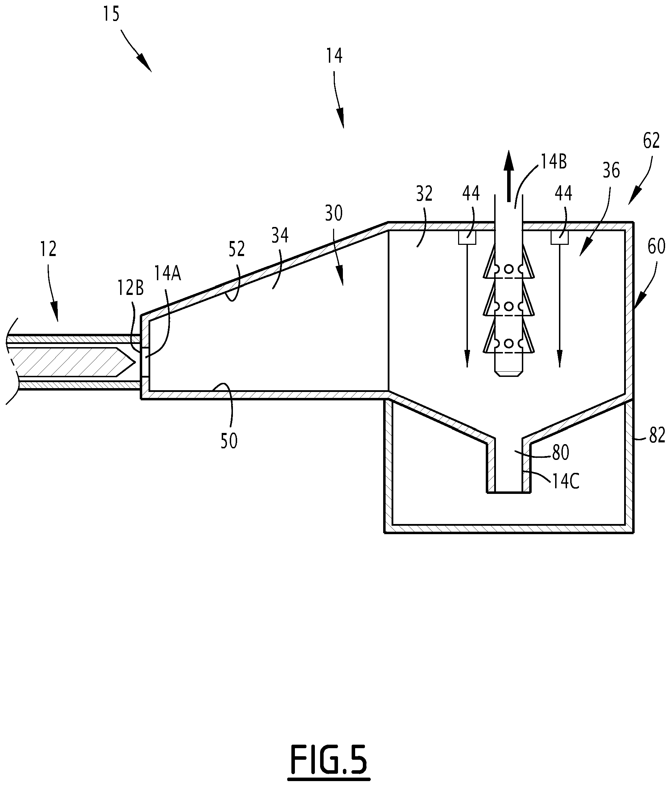

The separator 14 of Figure 5, on which numeral references to similar elements are the same, differs from that of Figure 2 - 4 namely in that the inlet duct 34 is delimited by a lower surface 50 that extends in a plane perpendicular to the central axis A and an upper surface 52 that is inclined to ascend from the upstream end of the inlet duct 34 to the downstream end of the inlet duct 34.

The separator 14 of Figure 5 differs from that of Figure 2 - 4 in that the spraying device 42 is arranged to spray the liquid monomer at a location proximate to the central axis A, in particular around the central axis A. The spraying device 42 comprises for example a plurality of spraying nozzles 44 distributed around the central axis A.

If a collection tube 36 is provided as illustrated on Figure 5, the spraying device 42 is for example configured for spraying the liquid monomer around the collection tube 36.

Spraying of liquid monomer retrieved from the condenser 16 into the separator 14 may be useful for preventing clogging of one or several demisting grids 48 provided inside the separator 14 over time, in particular by spraying the liquid monomer onto the demisting grids 48.

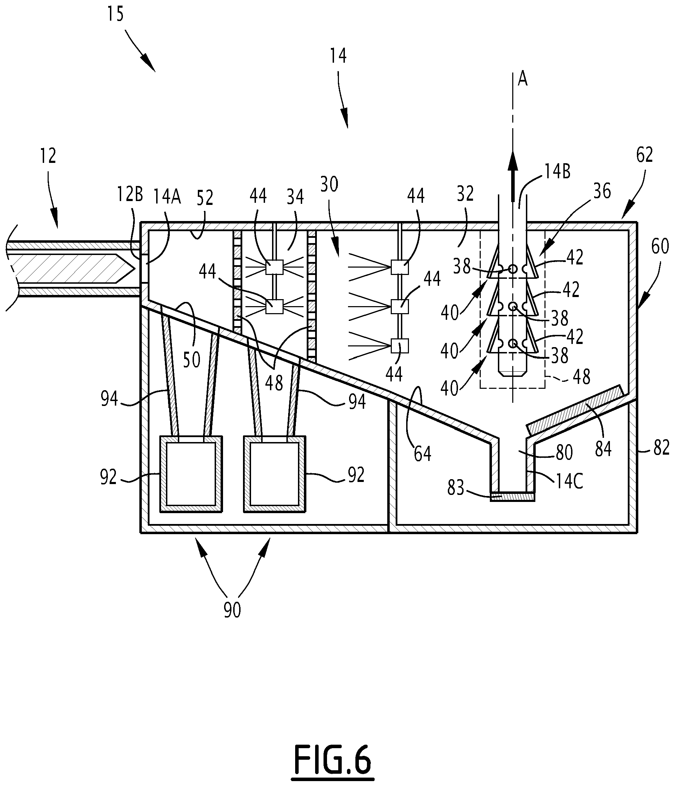

The separator 14 of Figure 6, on which numeral references to similar elements are the same, comprises spraying nozzles 44 orientated to spray liquid monomer retrieved from the condenser 16 onto the most downstream demisting grid 48 that is provided in the inlet duct 34 of the separator 14.

Optionally, as illustrated on Figure 6, the separator 14 comprises one or several spraying nozzles 44 arranged for spraying liquid monomer retrieved from the condenser 16 on a face of the most upstream demisting grid 48 that is provided in the inlet duct 34 of the separator 14.

Optionally, as illustrated on Figure 6, the separator 14 comprises one or several spraying nozzles 44 arranged for spraying liquid monomer retrieved from the condenser 16 in an intermediate space delimited between two demisting grids 48 that are provided in the inlet duct 34 of the separator 14, preferably with spraying liquid monomer onto one or both of these two demisting grids 48.

Optionally, as illustrated on Figure 6, the separator 14 comprises a demisting grid 48 provided in the downstream section of the separator, in particular a demisting grid 48 that surrounds the collecting tube 36.

This demisting grid 48 is, for example, arranged in a tubular shape and fitted around the collecting tube 36 such that the gas stream has to pass through this demisting grid to attain the collecting tube 36.

The gas stream entering the separator 14 may contain solid particles due to impurities of the raw material that is being depolymerized in the pyrolysis reactor 12.

Optionally, the separator 14 is provided with at least one solid particles collector 90 that is preferably arranged in the inlet duct 34 of the separator 14, preferably upstream a demisting grid 48 provided in the inlet duct 34 of the separator 14.

Each solid particles collector 90 comprises e.g. a collecting pot 92 connected to the separator 14 by a collecting duct 94 such that solid particles fall by gravity into the collecting pot 92. The collecting duct 94 connects for example to a lower wall 50 of the inlet duct 34 of the separator 14.

The collecting pot 92 is preferably removably connected to the separator 14. This allows emptying the collecting pot 92 when solid particles are collected therein or replacing the collecting pot 92 by a new one. The collecting pot can be equipped with one or two valves to isolate it from the separator 14, and allow the replacement of the collecting pots.

Preferably, each solid particles collector 90 is located in a climatic chamber 96 that is controlled to maintain therein a temperature equal to or above the boiling temperature of the monomer.

The separator 14 may be provided with one or several solid particles collectors 90.

As illustrated on Figure 6, the separator 14 is provided with two solid particles collectors 90 each connected to the inlet duct 34 of the separator 14 at a respective location along the inlet duct 34 of the separator 14.

When a plurality of solid particles collectors 90 are provided, each solid particles collector may be connected to the inlet duct 34, of the separator 14, upstream a respective one of a plurality of demisting grids 48 provided in the inlet duct 34 of the separator 14.

As illustrated on Figure 6, the separator 14 is provided with two demisting grids 48 extending across the inlet duct 34 of the separator 14 and with two solid particles collectors 90 each connected to the inlet duct 34 of the separator 14 upstream a respective one of these two demisting grids 48.

One of the solid particles collectors 90 is connected to the inlet duct 34 upstream the first demisting grid 48, and one of the solid particles collectors 90 is connected to the inlet duct 34 between two demisting grids 48.

When a plurality of solid particles collectors 90 are provided, they can be housed in the same climatic chamber 96.

As illustrated on Figure 6, the separator 14 is provided with two solid particles collectors 90 arranged in the same climatic chamber 96.

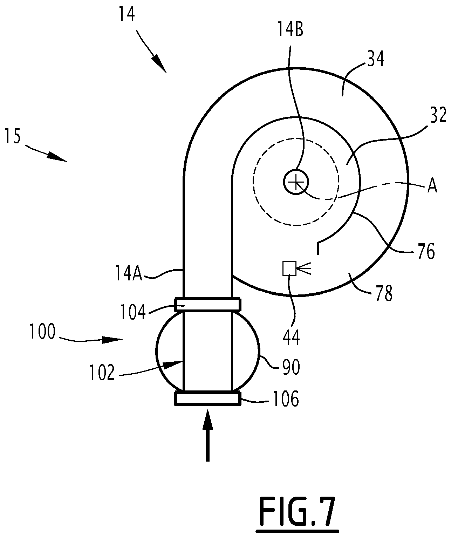

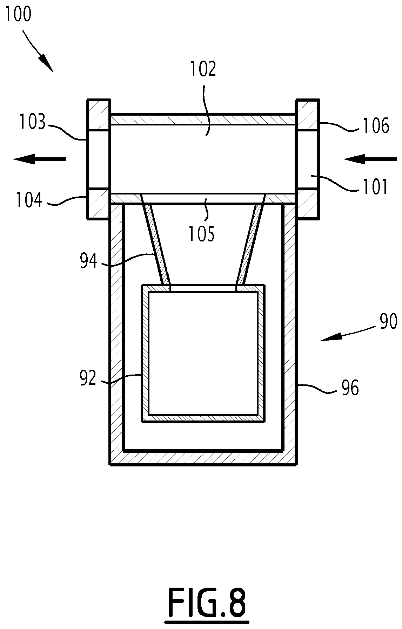

As illustrated on Figure 7 and 8, advantageously, the separator unit 15 comprises a primary separator 100 configured for separating dust from the gas stream generated by the pyrolysis reactor 12 before the gas stream enters the separator 14.

The primary separator 100 comprises a separation cavity 102 having a gas inlet 101 to be connected to the pyrolysis reactor 12, a gas outlet 103 to be connected to the gas inlet 14A of the separator 14 and a dust outlet 105 for collecting the dust.

As illustrated on Figure 7 and 8, the gas inlet 101 is for example coaxial with the gas outlet 103, such that in operation the gas flows along a straight line from the gas inlet 101 to the gas outlet 103, the dust outlet 105 being formed in a bottom wall of the separation cavity 102.

A solid particle collector 90 is connected to the dust outlet 105 for collecting dust.

In operation, dust that is too heavy to flow with the gas stream through the separation cavity 102 falls down in the dust outlet 105 and is collected in the solid particle collector 90.

The gas inlet 101 is for example provided with a first flange 104 for removable connection to the separator 14 and the gas outlet 103 is for example provided with a second flange 106 for removable connection to pyrolysis reactor 12.

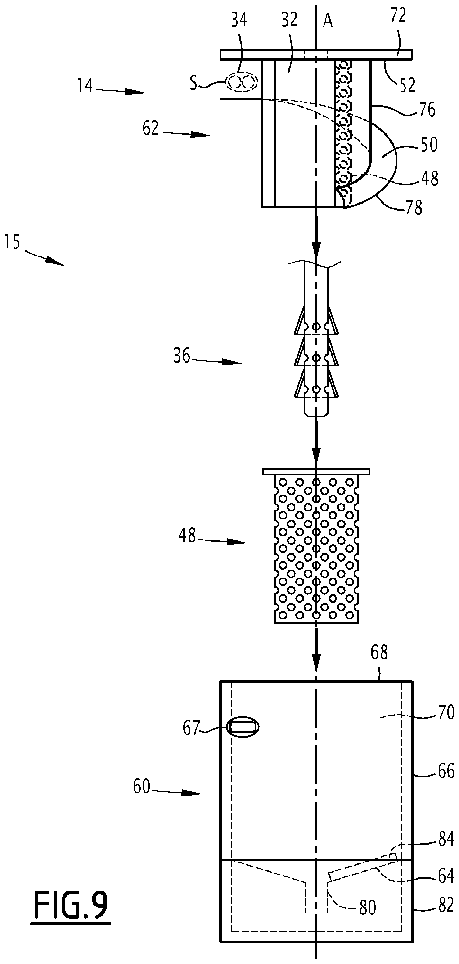

As illustrated on Figure 9, the separator 14 may comprise a demisting grid 48 extending across the internal flow passage 30 at the junction between the inlet duct 34 and the chamber 32.

The demisting grid 48 is for example provided on the top part 62 with being attached on the partition wall 76 and the duct element 78.

Preferably, the demisting grid 48 is arcuate such that the demisting grid is bulging in the upstream direction. The demisting grid 48 has for example two rectilinear vertical side edges and a top edge and a bottom edge that are arcuate. This provides a larger free cross-section for the passage of the gas through the demisting grid 48.

As illustrated on Figure 9, a tubular demisting grid 48 is also provided inside the chamber 32 and around the collecting tube 36 to cover the collecting tube 36.

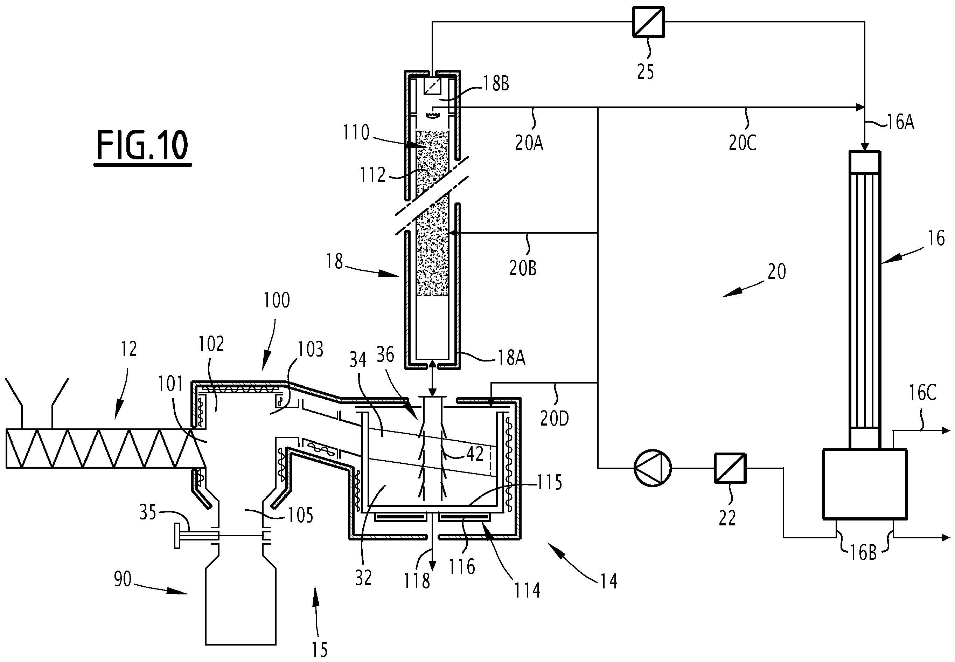

As illustrated on Figure 10, in an alternative example of the primary separator 100, the separation cavity 102 is configured for forcing the gas stream to change direction at least one time between the gas inlet 101 and the gas outlet 103 of the separation duct 102 such as to promote separation of the dust.

Advantageously, the separation cavity 102 is configured for the gas stream to enter horizontally in the gas inlet 101 and to be force to change direction to flow upwardly towards the gas outlet 103, the dust outlet 105 being provided at a bottom of the separation cavity 102.

The change of direction of the gas stream in the separation cavity 102 promotes separation due to the interaction of the dust particles, which tend to hit the side walls of the separation cavity 102 and to fall down in the dust outlet 105 provided at a bottom of the separation cavity 102.

As indicated above, the primary separator 100, and in particular the separation cavity 102, has to be taken into account for determining the ratio between the largest cross-section of the internal passage of the separator unit 15 (including the separator 14 and, if applicable, the primary separator 100) and the maximum flow rate of the gas stream provided by the pyrolysis reactor 12 and for determining the residence time of the gas stream in the separator unit 15.

A transfer column 18 suitable for implementing the depolymerization method is illustrated in Figure 10.

The transfer column 18 comprises a lower end 18A fluidly connected to the gas outlet 14B of the separator 14 for receiving the gas stream exiting the gas outlet 14B of the separator 14 and an upper end 18B fluidly connected to the gas inlet 16A of the condenser 16.

The depolymerization system 10 comprises the return line 20A fluidly connecting the condenser 16 to the upper end 18B of the transfer column 18 for injecting liquid monomer into the upper end 18B, in this example via a filtering device 22.

Optionally, one or several intermediate injection return lines 20B are provided for injecting liquid monomer in the transfer column 18 at one or several injection points located between the lower end 18A and the upper end 18B of the transfer column 18.

The lower end 18A of the transfer column 18 is fluidly connected to the separator 14, such that liquid collected at the lower end 18A of the transfer column 18 is transferred to the separator 14, in particular to the separation chamber 32 and/or to the inlet duct 34 of the separator 14.

The transfer column 18 comprises internals 110 configured such that, in operation, the gas stream flow upwardly from the lower end 18A to the upper end 18B, the cold monomer injected in the transfer column 18 flows downwardly inside the transfer column 18 by gravity, heavy contaminants contained in the gas stream condense in liquid state on the internals 110 and flow back down by gravity towards the lower end 18A of the transfer column 18 with being then transferred to the separator 14, and the temperature of the gas stream decreases from the lower end 18A to the upper end 18B of the transfer column 18.

The function of the internals 110 provided in the transfer column 18 is to increase the contact surface area for the gas stream flowing upwardly in the transfer column 18 and the liquid stream flowing downwardly in the transfer column 18

The internals 110 comprise for example a packing 112. The packing 112 may comprise random packing and/or structured packing.

Known types of random packing and structured packing are described for example on the web page https://chemiopedia.com/packed-bed-column-and-its-types.

The internals 110 comprises e.g. random packing, the random packing including for example so-called rings and/or saddles. The rings and/or saddles of the internals 110 are preferably made of metal or ceramic. The use of metal or ceramic allows withstanding the temperatures encountered in the transfer column 18.

The internals 110 comprise for example random packing including or consisting of rings, in particular so-called Pall rings. Rings, in particular Pall rings, offer a good interaction between gas and liquid.

Structured packing comprises for example alternating layers of mesh, gauze or fine corrugated sheets.

Known packing providers are for example the companies Sulzer, Montz and Koch-Glitsch Intalox. The internals 110 of the transfer column 18 may comprises different types of packing in respective sections of the transfer column 18. The internals 110 may comprise for example a first packing of a first type in a first section of the transfer column 18 and a second packing of a second type in a second section of the transfer column 18, the first type being different from the second type and the first section being distinct from the second section.

In particular, it is advantageous to tune the internals 110 of the transfer column 18 such as to impart to the transfer column 18 a higher fouling resistance in a lower section of the transfer column 18 as compared to an upper section of the transfer column 18, and to provide an improved distribution at the upper section of the transfer column 18 as compared to the lower section of the transfer column 18.

In addition, the pyrolysis reactor 12 is represented on Figure 10 as a heated screw extruder and the separator 14 is represented with a helical inlet duct 34 and a collection tube 36 provided with baffles 42 and extending in the separation chamber 34, the collection tube 36 being fluidly connected to the lower end of the transfer column 18 for feeding the transfer column 18 with the gas stream.

These features are optional and exemplary and the depolymerization system 10, in particular the pyrolysis reactor 12 and the separator 14, may be according to the examples and variants as discussed above.

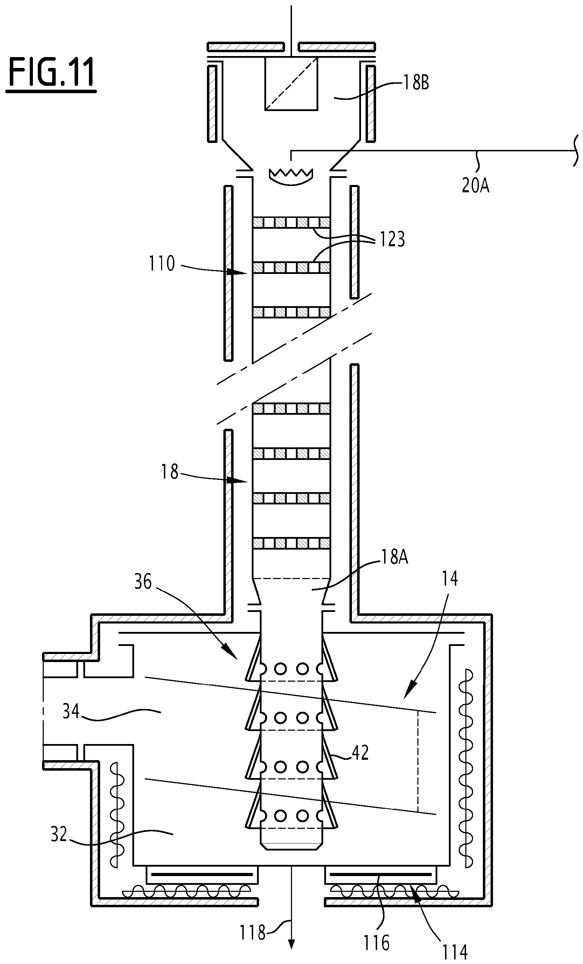

The transfer column 18 of Figure 11 differs from that of Figure 10 in that the internals 110 comprise a succession of filters 123 distributed along the transfer column 18, each filter 123 being provided with or delimiting holes are arranged inside the transfer column 18 for forcing circulation the gas stream and the liquid through said holes.

Each filter 123 is for example flat or in the shape of a cylinder. Each filter 123 is for example provided as a grid, a perforated plate or a mesh.

Such internals 110 force the gas stream and the liquid stream to circulate via the holes of the filters 123 with being in contact with each other.

As illustrated on Figure 11, the lower end 18A of the transfer column 18 is for example connected to a collecting tube 36 of the separator 14, such that the gas stream exiting the separator 14 is circulated directly to the transfer column 18 and the liquid flowing downwardly in the transfer column 18 is returned to the separator 14 via the collecting tube 36.

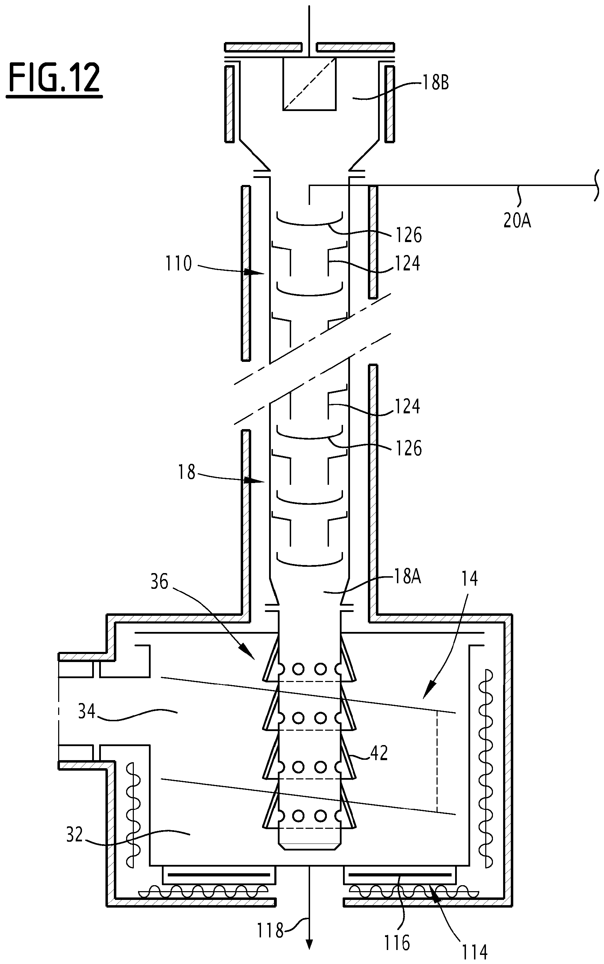

The transfer column 18 of Figure 12 differs from that of Figure 10 in that the internals 110 comprise a succession of plates or baffles distributed along the transfer column 18.

The baffles are configured to force the gas stream flowing upwardly and the liquid stream flowing downwardly to be in contact. In particular, the baffles are preferably configured for forcing the gas stream and the liquid stream to follow crossing tortuous paths.

The baffles comprises for example first baffles 124 alternating with second baffles 126 along the transfer column 18, each first baffle 124 being annular and delimiting a central passage for the gas stream and the liquid stream, each second baffle 126 being circular and delimiting an annular passage of the gas stream and the liquid stream, the inner diameter of the first baffles 124 being preferably smaller than the outer diameter of the second baffles 126.

Such first baffles 124 and second baffles 126 force the gas stream to flow through the liquid streams that flow by gravity with falling from the edges of the first baffles 124 and second baffles 126.

The depolymerization method optionally comprises inputting thermal energy inside the transfer column 18 and/or in the separator 14 by heating the gas stream and/or liquid present inside the transfer column 18 and/or inside the separator 14.

Inputting thermal energy notably enables controlling the temperature ranges of the gas stream in the separator 14 and in the transfer column 18.

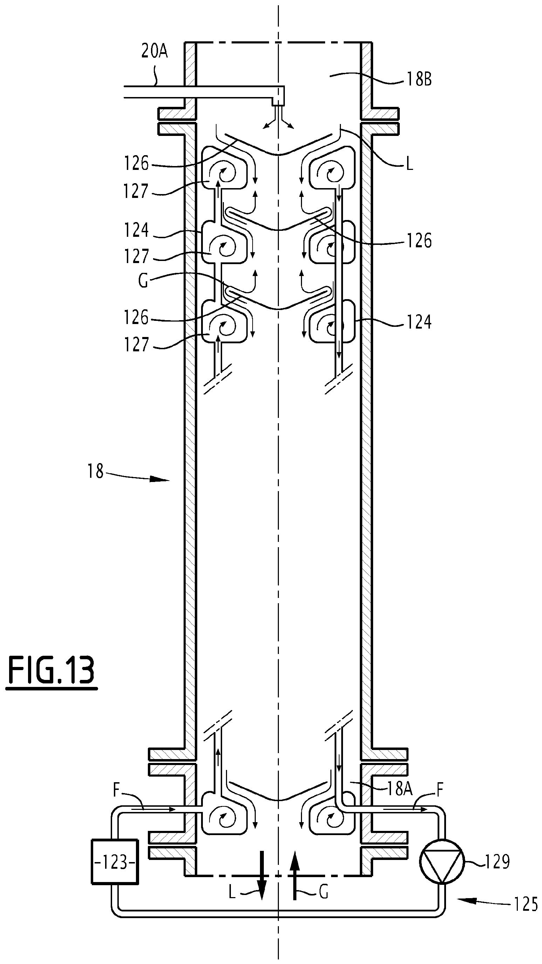

As illustrated on Figure 13, the transfer column 18 is for example configured for inputting thermal energy by heating the gas and liquid stream circulating in the transfer column 18.

The transfer column 18 of Figure 13 differs from that of Figure 12 in that at least part of the baffles are configured for heating of these baffles, named in the following "heated baffles".

The heated baffles are for example jacketed, i.e. provided with internal conduits for circulation of a heating fluid F.

As illustrated on Figure 13, the first baffles 124, which are annular, are heated baffles, in particular jacketed baffles. Optionally or alternatively, the second baffles 126, which are circular, are heated baffles, in particular jacketed baffles.

Heated baffles are provided in the transfer column 18 all along the transfer column 18 or only in a section of the transfer column 18.

As illustrated on Figure 13, heated baffles are provided all along the transfer column 18. Alternatively, heated baffles are provided only in a lower section of the transfer column 18, an upper section of the transfer column 18 comprises solely non-heated baffles or packing.

The jacketed heated baffles comprise internal ducts 127 for circulation of a heating fluid F. Each jacketed baffle 124 is for example hollow with delimiting an internal duct 127 inside the hollow jacketed baffle 124 for circulation of the heating fluid F.

The heating fluid F circulates preferably in closed loop in a heating circuit 123 including the jacketed baffles and further comprising a heater and/or a heat exchanger 125 and a pump 129 for heating the heating fluid F.

The circulations of the gas stream flowing from the lower end 18A to the upper end 18B and of the liquid monomer flowing from the upper end 18B to the lower end 18A are illustrated respectively by arrows G and L on Figure 13.

Internals 110 of the transfer column 18 may comprise one type of internals 110 such as a packing 112 (Figure 10), perforated plates or grids (Figure 11), baffles (Figure 12), possibly including or consisting in heated baffle (Figure 13).

Internals 110 of the transfer column 18 may comprising a combination of different types of internals 110, with each type of internals 110 being e.g. located in a respective section of the transfer column.

Optionally, the depolymerization method comprises heating liquid present or accumulating inside the separator 14 above the boiling temperature of the monomer such as to evaporate the monomer present in this liquid, the liquid being heated inside the separator 14 such as to evaporate the monomer inside the separator and/or heated outside the separator 14, the evaporated monomer being returned to the separator 14 such that the evaporated monomer flows with the gas stream circulating inside to separator 14.

The liquid present in the separator 14 provides from the transfer column 18 or from condensation inside the separator 14, e.g. on surfaces of the separator 14.

At least partly evaporating the liquid retrieved form the transfer column 18 or condensed in the separator 14 is also known as "reboiling" or "reheating".

Correspondingly, the depolymerization system 10 optionally comprises a reheating device 114 configured for evaporating liquid present and/or accumulating in the separator 14, the reheating device 114 being configured such that the gas resulting from evaporation is generated in the separator 14 and/or returned to the separator 14 and thus flows with the gas stream to the transfer column 18.

As illustrated on Figure 1, the depolymerization system 10 comprises for example a heating device 114 configured for heating the liquid outside the separator 14 and reinjecting the gas resulting from the reheating inside the separator 14.

The heating device 114 comprises a heater 116 arranged outside the separator 14, the heater 116 reheats the liquid collected from the separator 14.

The depolymerization system 10 comprises for example a reheating loop 111 configured for collecting liquid from the separator 14, heating the liquid in the reheater 116 such as to obtain a gas fraction and reinjecting the gas and liquid resulting from the reheating in the separator 14.

In stable operating conditions, most of the monomer in liquid state that condenses in the separator 14 or arrives in liquid state from the transfer column 18 will reevaporate in the reheater 116, so that there is no monomer accumulation.

Optionally, the reheating loop 111 comprises a filtering device 113. The filtering device comprises for example one filter or two filters connected fluidly in parallel.

Preferably, the reheating loop 111 comprises a pump 144 for forcing the fluid circulation in the reheating loop 111.

Optionally, the depolymerization system 10 comprises a liquid monomer reservoir 117 for feeding liquid monomer in the reheating loop 111, e.g. during a starting phase and/or a cleaning phase.

Preferably, the depolymerization system 10 comprises a collecting reservoir 119 connected to the reheating loop 111, preferably via a heated line 121, for collecting heavies accumulating in the reheating loop 111, from time to time.

As illustrated on Figure 10 - 14, a reheating device 114 is for example configured for heating the liquid inside the separator 14.

The reheating device 114 is for example configured for heating liquid collected on a collecting surface 115 of the separator 14 on which the liquid present in the separator 14 collects by gravity.

The collecting surface 115 is for example a bottom surface of the separator 14 on which liquid present in the separator 14 is collected, in particular the bottom of the separation chamber 32 of the separator 14.

As illustrated on Figures 10 - 14, the reheating device 114 comprises an electrical heater 116 arranged outside the separator 14 along the collecting surface 115 of the separator 14 on which liquid present in the separator 14 is collected by gravity.

Preferably, the depolymerization system 10 comprises a drainage device 118 configured for draining heavy liquids accumulating on the collecting surface 115 of the separator 14 on which liquid present in the separator 14 is collected by gravity.

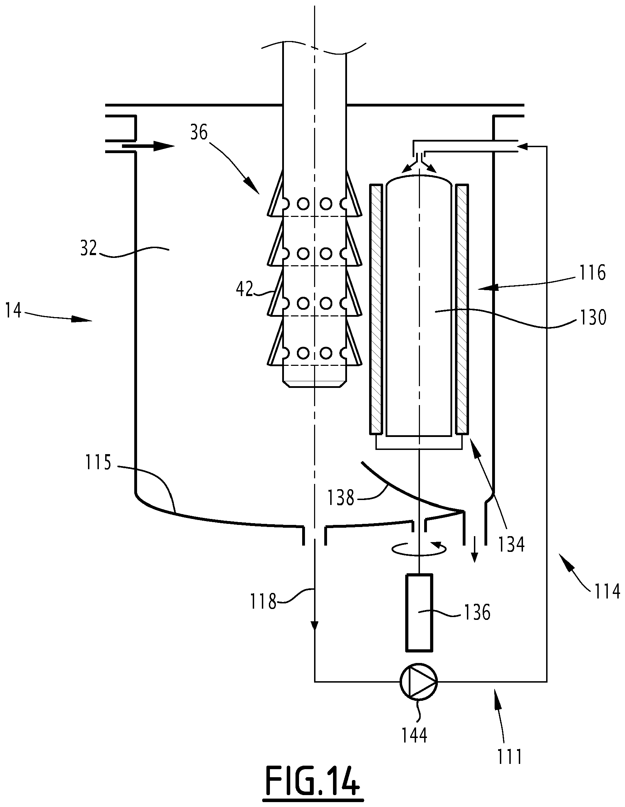

As illustrated on Figure 14, the reheating device 114 comprises a reheater 116 installed inside the separator 14, in particular in the chamber 32 of the separator 14, and configured for evaporating liquid present in the separator 14.

The reheater 116 is for example an evaporator, in particular a wiped film evaporator comprising a hollow cylinder 130 extending vertically and configured for receiving hot oil inside the cylinder 130, a reheating loop 111 configured for feeding liquid collected in the separator 14 onto the upper end of the hollow cylinder 130 such as to form liquid film flowing by gravity on the external surface of the cylinder 130, a rotary wiper 134 coupled to a driving motor 136 for rotating the rotary wiper 134 around the cylinder 130 and wiping the liquid film onto the outer surface of the cylinder 130.

The reheating device 114 comprises preferably a collector 138 for collecting the liquid dropping from the cylinder 130 and evacuating this liquid out of the separator 14, with avoiding this liquid to return to the collecting surface 115 of the separator 14.

The reheating device 114 allows evaporating liquid present in the separator 14 for retrieving any monomer that would still be present in this liquid and evacuating heavies outside of the separator 14.

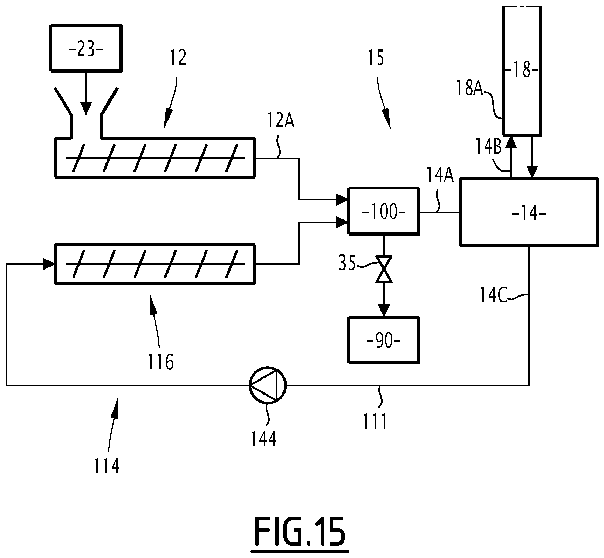

In one example, as illustrated on Figure 15, the reheating device 114 comprises a reheater 116 provided as screw extruder fluidly connected to the separator 14 for receiving the liquid collected in the separator 14 and extruding this liquid such as to at least partially evaporate the monomer and return the gaseous monomer to the separator 14, for example to the gas inlet 14A of the separator 14.

A screw extruder is capable of handling highly viscous liquids and produce a solid residue and gaseous monomer. The gaseous monomer is returned to the separator 14, while the solid/heavy residues are for example disposed.

Advantageously, as illustrated on Figure 15, the liquid retrieved from the separator 14 is fed to the reheater 116 provided as a screw extruder and the outlet of the reheater 116 provided as a screw extruder is connected to the separator 14, in particular to the gas inlet of the separator 14, e.g. via the primary separator 100.

The heater 116 of the reheating device 114 may be located outside the separator 14 for heating liquid present inside the separator 14 (Figure 10), or located outside the separator 14 for heating liquid collected from the separator 14 (Figures 1 and 15), or located inside the separator 14 (Figure 14).

Besides, different types of heater 116 may be provided, including for example extruders or evaporators, in particular thin film evaporators.

### EXAMPLES

Examples have been implemented in a depolymerization system 10 comprising a pyrolysis reactor 12, and separator 14, a condenser 16 and a transfer column 18.

The pyrolysis reactor 12 is provided as a twin-screw extruder with co-rotation of the screws and intermeshing of the screw.

The twin-screw extruder includes the screw extending through a barrel, an electric heater providing the heating energy, which is installed around the barrel, and thermocouples for temperature control which are also installed in the barrels. The barrel is for example formed by barrel segments.

The twin-screw extruder, and in particular its heaters, are configured for generating a temperature profile along the screws, with a maximum profile temperature at an intermediate section along the screws.

The twin-screw extruder is a TEX44 having a 47mm screw diameter. For industrial production line, a TEX90 having a 96.5mm screw diameter may be used according to the requested feeding rate. TEX extruders are marketed by The Japan Steel Works, Ltd which also offers larger units.

The material to be depolymerized is fed into the twin-screw extruder at a hopper barrel by gravimetric screw feeders. The material provided to the gravimetric screw feeders is previously crushed to a size of about 5 to 10 mm.

The twin-screw extruder has along its length a melting section followed by a depolymerization section.

In operation, the material is molten in the melting zone of the twin-screw extruded by shear stress from screw and heat from the heaters, then the melted material is transported to the depolymerization section.

The depolymerization section of the pyrolysis reactor 12 can achieve a temperature of 550°C.

PMMA mixtures in the form of crushed particles were fed through the gravimetric feeders. Two to four independent feeders were loaded with cast, injection and/or extrusion PMMA.

The following operating conditions were used during comparative examples and the examples of the invention: a feed rate of the material in the pyrolysis reactor 12 comprised between 50 and 100 kg/h, a rotation speed of the screws of the pyrolysis reactor 12 of about 800 rotations per minute, and a maximum profile temperature in the pyrolysis reactor 12 of 470 °C or less.

For examples 1 and 2, the transfer between the separator 14 and the condenser 16 is performed via a transfer column 18 having a 5.08 cm inner diameter in which an internal provided as filter mesh is inserted. The filter mesh is a cylindrical grid with holes having a diameter of 1 mm. The filter mesh is inserted at an angle in the transfer column so that the gas stream is filtered through the filter mesh. The filter mesh is placed in the upper part of the transfer column 18, closer to the condenser 16 than to the separator 14.

### Example 1 (comparative):

In example 1, the filter is operated "dry" meaning without liquid cold monomer (MMA) injecting in the transfer column.

End-of-Life car tail lights PMMA, collected at car deconstruction site, and crushed to small particles was used. It contains mostly PMMA and some contamination with other polymers like ABS. The average age of a car arriving in a deconstruction site is about 17 years, so the scraps contain all kind of PMMA, pigments and additives from all past and present suppliers.

The feed rate of the injection scraps was 50 kg/h, the maximum temperature of pyrolysis reactor 12 extruder was set to 470 °C, with a screw rotation speed of 800 rotations per minute, and the depolymerization system 10 operated at atmospheric pressure.

The color of the crude MMA product collected was deep red. The color was measured according to the ASTM D1500 - 07 standard method. The value recorded was L3.0 ASTM Color.

### Example 2 (according to the invention):

The previous example is continued but 10 kg/h of liquid cold monomer (MMA) is injected at the upper end of the transfer column 18, through the filter mesh and towards the separator 14. The other operating parameters remained the same.

The color of the crude MMA product collected at the outlet of the condenser 16 was deep red. The color was measured according to the ASTM D1500 - 07 standard method. The value recorded was L2.5 ASTM Color.

The temperature of the gas at the gas inlet 16A of the condenser 16 dropped by 51 °C as compared to the comparative example 1. This illustrates that the liquid MMA sent to the transfer column 18 has a cooling effect.

In addition, after the test, the filter was checked. It was clean, unlike in example 1.

In both examples 1 and 2, the temperature of the MMA at the outlet of the condenser bottom remained between 12 and 13 °C.

The table below reports the analysis of the samples by gas chromatography. The major difference detected is in the heavy compounds (determined by GC based on the retention time). The products collected at MMA retention time + 2 minutes and above, are much higher in the example 1 case than in the example 2 case.

| SAMPLES | **Example 1** | **Example 2** |
|---|---|---|
| Feed rate (kg/h) / Screw rotation (rpm) | 50 / 800 rpm | |
| Temperature (°C) | 470 °C | |
| Conditions | 1.0 Bars | 1.0 Bars |
| Clear = without particles | clear red | **clearer red** |
| GC/FID with internal Standard on a polar column | | |
| **MMA (wt%) (+/-2%)** | **91** | **93** |
| ethyl acrylate (wt%) | 0.25 | 0.26 |
| methyl isobutyrate (wt%) | 0.20 | 0.22 |
| | | |
| GC/FID on apolar column (area%) | | |
| unknown heavies (Tr MMA+2min)-(Tr MMA+8min) | 1.57 | 1.31 |
| unknown very heavies > (Tr MMA+8min) | 3.62 | 2.18 |

The depolymerization method and the corresponding depolymerization system 10 for implementing the depolymerization method allows producing a liquid monomer from a feed material containing the corresponding polymer, with obtaining a satisfying purity and maximizing the running time of the depolymerization system 10.

Indeed, providing a separator 14 allows trapping impurities that may be present in the gas stream and avoids these impurities clogging the components of the depolymerization system 10 that are located downstream the separator 14.

Providing the transfer column 18 keeps the gas stream arriving to the condenser 16 clean, with trapping impurities in the cold monomer flowing by gravity in the transfer column 118 and returning these impurities to the separator 14. The transfer column 18 is backwashed with cold monomer thus limiting the risk of clogging.

Input of thermal energy in the separator 14 (e.g. via the heating device 114) and/or the transfer column 118 (e.g. via the heated baffles) avoids wasting to much cold monomer due to the circulation of the cold monomer in the transfer column 18 for trapping impurities present in the gas stream.

The separator 14 can be used as a reheater for boiling the liquid that is returned to the separator 14, in particular when the separator 14 is provided with a reheating device 114 including a heater 116.

Inputting thermal energy in the separator 14 and/or the transfer column 18 allows increasing the amount of cold monomer that is injected in the transfer column 18 and thus decreasing the risk of clogging of the transfer column 18.

Operating the separator 14 and the transfer column 18 in specific temperature ranges allow avoiding any condensation and polymerization of the monomer in the separator 14 of the transfer column 18 and promotes a good operation of the separator and the transfer column 18. This further allows spraying cold liquid monomer inside the separator 14 for controlling the temperature of the gas stream inside the separator 14.

The quality of the cold monomer that is eventually obtained in increased, with less impurities, less dimer, less trimer and less wax present in the cold monomer.

The depolymerization system can operated with longer cycle times between cleaning operations.

Some specific arrangements of the separator 14 and the transfer column 18, such as the limited linear velocity of the gas stream, the separation chamber 32, the inlet duct 14 having a varying cross-section and/or extending around the separation chamber 32, the collection tube 36 with a specific structure and/or the internal demisting device 46, the internals of the transfer column 18 and/or the injection of cold monomer retrieved from the condenser 16 into the transfer column 18, allow trapping the impurities efficiently while managing temperature of the gas stream and allowing the gaseous monomer to flow to the separator outlet 14B for feeding the condenser 16 via the transfer column 18.

## Claims

1. Depolymerization method for recovering a monomer from a polymer, the depolymerization method comprising the successive steps of pyrolizing a feed material containing the polymer in a pyrolysis reactor (12) so as to generate a hot gas stream, removing solid and liquid impurities from the gas stream in a separator (14) and condensing the monomer contained in the gas stream in a condenser (16),
wherein the gas stream is transferred from the separator to the condenser via a transfer column (18) extending upwardly from a lower end fluidly connected to an outlet of the separator (14) to an upper end fluidly connected to an inlet of the condenser (16),
wherein cold monomer in liquid state is injected into the transfer column (18), the transfer column being provided with internals configured such that the cold monomer flows downwardly inside the transfer column (18) by gravity, heavy contaminants contained in the gas stream condense in liquid state on the internals and flow back down by gravity towards the bottom of the transfer column (18) with being then transferred to the separator (14) and the temperature of the gas stream decreases from the lower end to the upper end of the transfer column (18), and
wherein the temperature of the gas stream at the outlet of the separator (14) and the inlet of the transfer column (18) is maintained above the boiling point of the monomer and the temperature of the gas stream at the outlet of the transfer column (18) is maintained between the boiling point of the monomer and the boiling point of the monomer + 50°C.

2. Depolymerization method according to claim 1, wherein the temperature of the gas stream at the outlet of the separator (14) and entrance of the transfer column (18) is maintained at least at the boiling point of the monomer + 20°C, in particular at least at the boiling point of the monomer + 30°C, more in particular at least at the boiling point of the monomer + 40°C, even more in particular at least at the boiling point of the monomer + 50°C.

3. Depolymerization method according to claim 1 or 2, wherein the temperature of the gas stream at the outlet of the transfer column (18) is maintained between the boiling point of the monomer and the boiling point of the monomer + 50°C, in particular between the boiling point of the monomer and the boiling point of the monomer + 40°C, in particular between the boiling point of the monomer and the boiling point of the monomer + 30°C, in particular between the boiling point of the monomer and the boiling point of the monomer + 20°C, in particular between the boiling point of the monomer and the boiling point of the monomer + 10°C.

4. Depolymerization method according to any one of the preceding claims, wherein the internals comprise one mesh filter or a plurality of mesh filters arranged in cascade inside the transfer column.

5. Depolymerization method according to any one of the preceding claims, wherein the internals comprise a structured packing and/or a random packing, for example a random packing including saddles and/or rings, in particular Pall rings.

6. Depolymerization method according to any one of the preceding claims, wherein the internals comprise a succession of baffles.

7. Depolymerization method according to any one of the preceding claims, wherein the internals comprise a succession of baffles comprising internally heated baffles, preferably alternating with non-heated baffles.

8. Depolymerization method according to any one of the preceding claims, wherein cold monomer in liquid state is injected at the upper end of the transfer column and/or at one or several intermediate locations between the lower end and the upper end of the transfer column.

9. Depolymerization method according to any one of the preceding claims, comprising the step of filtering the cold monomer before injection of the cold monomer into the transfer column (18).

10. Depolymerization method according to any one of the preceding claims, comprising a step of inputting thermal energy inside the transfer column (18) and/or in the separator (14) by heating the gas and/or the liquid present inside the transfer column (18) and/or inside the separator (4).

11. Depolymerization method according to any one of the preceding claims, comprising heating liquid collected in the separator (14) using a heater located inside the separator and/or heater located outside the separator (14) for evaporating monomer contained in the liquid and returning the monomer to the separator (14).

12. Depolymerization method according to claim 10, wherein heating of the liquid is performed:
- with a heater provided as an electrical heater arranged outside the separator (14) for heating liquid collected inside the separator (14);
- with a heater provided as an evaporator, in particular a wiped film evaporator, preferably an evaporator arranged inside the separator, the evaporator being fed with liquid collected from the separator; and/or
- with a heater provided as a heated screw extruder fed with liquid collected from the separator (14), a gas produced by the heater provided as a heated screw extruder being fed to the separator (14).

13. Depolymerization method according to any one of the preceding claims, wherein the rate of cold monomer injected in the transfer column (18) is at least 50%, preferably at least 60%, preferably at least 70% preferably at least 80%, preferably at least 90% of the equivalent feed rate of the monomer.

14. Depolymerization method according to any one of the preceding claims, wherein the fraction of cold monomer injected in the transfer column (18) is less than 153%, preferably less than 103%, preferably less than 93% preferably less than 83%, preferably less than 73%, preferably less than 63%, preferably less than 53% of the equivalent feed rate of the monomer.

15. Depolymerization method according to any one of the preceding claims, wherein the temperature of the gas stream at the inlet of the separator (14) is equal or higher than 250°C.

16. Depolymerization method according to any one of the preceding claims, wherein the temperature of the gas stream at the outlet of the separator (14) is comprised between 100 °C and 280 °C, preferably between 110°C and 180°C.

17. Depolymerization method according to any one of the preceding claims, wherein the pressure inside at least one or each of the pyrolysis reactor (12), the separator (14) and the condenser (16) is comprised between 0.3 to 2.0 bars, preferably between 0.4 and 1.5 bars, more preferably between 0.5 and 1.2 bars.

18. Depolymerization system configured for recovering a monomer from a polymer, the depolymerization system comprising a pyrolysis reactor (12) configured for pyrolizing a feed material containing the polymer so as to generate a gas stream, a separator (14) fluidly connected to the pyrolysis reactor (12) for receiving the gas stream from the pyrolysis reactor (12) and configured for removing impurities from the gas stream, and a condenser (16) fluidly connected to the separator (14) for receiving the gas stream from the separator and configured for condensing the monomer contained in the gas stream received from the separator (14),
wherein the depolymerization system comprises a transfer column (18) for transferring the gas stream from the separator (14) to the condenser (16), the transfer column (18) extending upwardly from a lower end fluidly connected to an outlet of the separator to an upper end fluidly connected to an inlet of the condenser (16), the transfer column (18) being configured for injection of cold monomer in liquid state inside the transfer column (18) and provided with internals configured such that in operation the cold monomer flows downwardly inside the transfer column (18) by gravity, heavy contaminants contained in the gas stream deposit in liquid state on the internals and flow back by gravity towards the separator (14) and the temperature of the gas stream increases from the lower end to the upper end of the transfer column (18).
